(19) Europäisches Patentamt
European Patent Office
Office européen des brevets

(11) EP 4 435 910 A1

(12) **EUROPEAN PATENT APPLICATION**
published in accordance with Art. 153(4) EPC

(43) Date of publication:
**25.09.2024 Bulletin 2024/39**

(21) Application number: **22894531.7**

(22) Date of filing: **14.10.2022**

(51) International Patent Classification (IPC):
***H01M 8/18** (2006.01)*

(52) Cooperative Patent Classification (CPC):
**H01M 8/18; H01M 8/2455;** Y02E 60/50

(86) International application number:
**PCT/CN2022/125238**

(87) International publication number:
**WO 2023/087993 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.11.2021 CN 202111356081**

(71) Applicant: **Westlake University**
**Hangzhou, Zhejiang 310024 (CN)**

(72) Inventors:
- **WANG, Jianhui**
  **Hangzhou, Zhejiang 310024 (CN)**
- **LI, Xinyu**
  **Hangzhou, Zhejiang 310024 (CN)**
- **LI, Hongju**
  **Hangzhou, Zhejiang 310024 (CN)**

(74) Representative: **Petraz, Gilberto Luigi et al**
**GLP S.r.l.**
**Viale Europa Unita, 171**
**33100 Udine (IT)**

# (54) ION EXCHANGE LIQUID FILM FLOW BATTERY

(57) The invention relates to an ion exchange liquid membrane flow battery. The ion exchange liquid membrane separates the catholyte and anolyte by independently stratifying with the catholyte and anolyte, it dissolves the supporting solute but does not dissolve active material in the catholyte and anolyte. The present invention uses the ion exchange liquid membrane to replace the traditional ion exchange solid membrane, solving the problems of high cost and short life of the latter. In addition, the technical solution of the present invention has significant advantages in preventing the crossover of active material due to the adjustable thickness of the ion exchange liquid membrane. Moreover, the technical solution of the present invention can flexibly match all kinds of acidic, neutral, alkaline aqueous or organic catholyte with anolyte according to application requirements, solving the problem of limited catholyte-anolyte match in a conventional flow battery. Furthermore, the technical solution of the present invention is advantageous for multi-cell assembly and large-scale application as the ion exchange liquid membrane has no fixed shape.

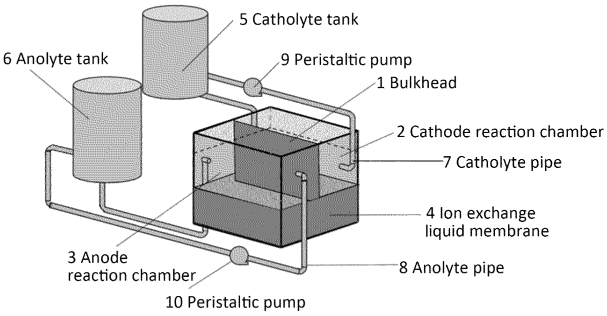


Fig.1

EP 4 435 910 A1

## Description

### Technical field

The present invention belongs to the technical field of flow batteries, and specifically relates to an ion exchange liquid membrane flow battery.

### Background Art

A key challenge facing human society in the 21st century is how to deal with the contradiction between growing energy consumption and global warming. It is a key way to overcome related challenges to use renewable energy sources such as solar and wind energy to replace fossil fuels. However, the large-scale use of solar energy and wind energy will impact the stability and security of the power system, since they are intermittent energy sources. Therefore, there is an urgent need to develop large-scale energy storage systems to achieve large-scale access of renewable energy, to achieve peak load shaving, to solve the problem of wind and power curtailment, and to improve the energy efficiency, stability and security of new power systems.

Currently, the research and development of large-scale energy storage technology is still in the exploratory and preliminary stages. Local small-scale attempts mainly choose lithium-ion battery energy storage technology. However, it is difficult for lithium-ion batteries to be large in size, because large-scale energy storage needs to manage millions or more batteries, which greatly increases the managing difficulty of energy storage system and its risks in safety and stability. Therefore, it is necessary to develop large-scale energy storage device which is economical, safe, and easy-to-manage.

Theoretically, flow batteries have many advantages such as flexible structure, adjustable capacity, high safety, ultra-long cycle life, low cost, etc., making them an ideal choice for renewable energy storage devices. The current flow battery with relatively mature technology is the all-vanadium flow battery, but it still faces high cost problems, including the expensive Nafion ion exchange membrane (accounting for 20-40% of the overall battery cost) and expensive vanadium-based electrolyte, which seriously hinder its large-scale promotion and use.

In the modification of battery membranes, membrane-free flow batteries are one of the important directions. The key is to develop immiscible catholyte and anolyte to achieve automatic isolation of electrochemically active materials in the catholyte and anolyte. However, the existing membrane-free flow batteries commonly have challenging problems such as small selection range and difficult matching due to the immiscibility of catholyte and anolyte, and serious crossover of electrochemically active materials. For example, Paula Navalpotro et al. reported a membrane-free flow battery with a biphasic immiscible system (A membrane-free redox flow battery with two immiscible redox Electrolytes, Angew. Chem. Int. Ed. 2017, 56, 12460-12465), which, however, has shortcomings of crossover of the biphasic active materials and limited matching of the catholyte and anolyte. Guodong Li et al. reported a membrane-free flow battery using a mutual micible system (membrane-free Zn/MnO2 flow battery for large-scale energy storage, Adv. Energy Mater. 2020, 1902085), which, however, has shortcomings of severe self-discharge, limited surface energy density, and limited matching of catholyte and anolyte. Jintao Meng et al. reported a stirred membrane-free flow battery (A stirred self-stratified battery for large-scale energy storage, Joule 4, 953-966, April 15, 2020), which, however, has shortcomings of expensive electrolyte, limited matching of catholyte and anolyte, and difficult scale-up in device.

Therefore, there is still a need to further develop new flow batteries.

### Summary of the Invention

The inventor of the present invention conducted in-depth research on flow batteries, proposed the idea of using an ion exchange liquid membrane, and verified the feasibility of this solution through experiments, thus completing the present invention.

In one aspect, the invention provides a flow battery including an ion exchange liquid membrane, wherein the ion exchange liquid membrane separates catholyte and anolyte by independently stratifying with the catholyte and the anolyte, and it dissolves a supporting solute but does not dissolve cathode and anode active materials.

The independent stratifying of ion exchange liquid membrane with the catholyte and the anolyte means that when the catholyte or the anolyte is mixed with the ion exchange liquid membrane, the two cannot form a homogeneous phase, but will spontaneously stratify to form a biphasic system under the action of gravity according to the difference in density, and the two phases have an obvious interface. Therefore, the ion exchange liquid membrane can function as a separator to physically separate the catholyte and the anolyte so that the catholyte and the anolyte do not come into contact.

The expression that the ion exchange liquid membrane does not dissolve the cathode and anode active materials means that the ratio of the solubility of the cathode or anode active material in the catholyte or the anolyte to the solubility

in the ion exchange liquid membrane (that is, the ratio of the solubility of the cathode active material in the catholyte to the solubility in the ion exchange liquid membrane, and the ratio of the solubility of the anode active material in the anolyte to the solubility in the ion exchange liquid membrane) is greater than 10, preferably greater than 100, more preferably greater than 1000. There is no specific limit to the upper limit of the above solubility ratio because it is most preferred that the cathode or anode active material is completely insoluble in the ion exchange liquid membrane.

The ion exchange liquid membrane can dissolve a supporting solute and provide a pathway for the transport of the supporting solute ions between the catholyte and the anolyte.

The technical solution of the present invention using an ion exchange liquid membrane uses the ion exchange liquid membrane as a substitute for the traditional solid ion exchange membrane, thereby solving the problems of high cost and short life of the traditional ion exchange solid membrane. In addition, the technical solution of the present invention using an ion exchange liquid membrane has significant advantages in suppressing crossover because the thickness of the ion exchange liquid membrane is adjustable. Moreover, the technical solution of the present invention using an ion exchange liquid membrane can flexibly match all kinds of acidic, neutral, alkaline aqueous or organic catholyte with anolyte according to application, solving the problem of limited catholyte-anolyte match in a conventional flow battery. In addition, the technical solution of the present invention using an ion exchange liquid membrane is advantageous for multi-cell assembly and facilitates the large-scale application because the ion exchange liquid membrane has no fixed shape.

According to the present invention, the ion exchange liquid membrane may be ionic liquid-based, water-based or organic solvent-based liquid. Here, the ionic liquid-based, water-based or organic solvent-based liquid means that the liquid has an ionic liquid, water or an organic solvent as its main component.

The ion exchange liquid membrane may further contain an other component, such as a supporting solute contained for the supporting solute dissolved in the ion exchange liquid membrane; a salting-out functional additive added to assist or achieve stratification with the catholyte and the anolyte; a very small amount of the cathode and anode active materials dissolved in the ion exchange liquid membrane, but the present invention is not limited thereto. In addition, according to the requirements for preparing a flow battery, for example, in order to expand the electrochemical stability window, adjust the density or pH of the electrolyte, the solubility of the electrochemical active materials, etc., an other functional additive may be further added into the catholyte, the anolyte or the ion exchange liquid membrane, and therefore the functional additive may also be present in the ion exchange liquid membrane in a certain amount.

There is no particular restriction on the ionic liquid suitable for use in the ion exchange liquid membrane as long as it meets the above requirements and is suitable for use in batteries. In particular, the ionic liquid suitable for use in the ion exchange liquid membrane can be selected from: a hexafluorophosphate-containing ionic liquid, a tetrafluoroborate-containing ionic liquid, a perchlorate-containing ionic liquid, a bis(trifluoromethylsulfonyl)imide-containing ionic liquid, a bisfluorosulfonimide-containing ionic liquid, a trifluoroacetate-containing ionic liquid, a dinitrile amine-containing ionic liquid, a trifluoromethanesulfonate-containing ionic liquid, a thiocyanate-containing ionic liquid, and a mixture thereof, but the present invention is not limited thereto.

In particular, the ionic liquid may be one or more selected from the following compounds: 1-butyl-3-methylimidazolium hexafluorophosphate ($BMIMPF_6$), 1-ethyl-3-methylimidazolium tetrafluoroborate ($EMIMBF_4$), 1-propyl-3-methylimidazolium tetrafluoroborate ($PMIMBF_4$), 1-butyl-3-methylimidazolium tetrafluoroborate ($BMIMBF_4$), 1-ethyl-3-methylimidazolium perchlorate ($EMIMClO_4$), 1-propyl-3-methylimidazolium perchlorate ($PMIMClO_4$), 1-butyl-3-methylimidazolium perchlorate ($BMIMClO_4$), 1-ethyl-3-methylimidazolium bis(trifluoromethylsulfonyl)imide ($EMIMNTf_2$), 1-propyl-3-methylimidazolium bis(trifluoromethylsulfonyl)imide ($PMIMNTf_2$), 1-butyl-3-methylimidazolium bis(trifluoromethylsulfonyl)imide ($BMIMNTf_2$), etc.

There is no particular restriction on the organic solvent suitable for use in the ion exchange liquid membranes as long as it meets the above requirements and is suitable for use in batteries. In particular, the organic solvent suitable for use in the ion exchange liquid membranes can be selected from: a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound, and a mixture thereof, but the present invention is not limited thereto.

In particular, the organic solvent may be selected from trimethyl phosphate, triethyl phosphate, triphenyl phosphate, tris(2,2,2-trifluoroethyl) phosphate, N,N-dimethylformamide, N,N-dimethylacetamide, N-methylpropionamide, trifluoroacetamide, N-methyltrifluoroacetamide, N,N-dimethyltrifluoroacetamide, hexamethyldisiloxane, hexa(trifluoropropyl)disiloxane, trifluoropropylmethylcyclotrisiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, etc., and a mixture thereof.

According to the requirements for the relationship between the ion exchange liquid membrane and the catholyte

and the anolyte, that is, the ion exchange liquid membrane is independently stratified with the catholyte and the anolyte, does not dissolve cathode and anode active materials, but dissolves supporting solute. Appropriate catholyte and anolyte can be selected through routine experiments according to the type of the selected ion exchange liquid membrane, or an appropriate ion exchange liquid membrane can be selected through routine experiments according to the selected catholyte and anolyte. Therefore, the technical solution of the flow battery using an ion exchange liquid membrane of the present invention can flexibly match all kinds of acidic, neutral, alkaline aqueous or organic catholyte and anolyte according to the application, solving the problem of limited catholyte-anolyte match in conventional membrane-free flow batteries.

In an embodiment, a flow battery according to the present invention includes:

an ion exchange liquid membrane, which is an ionic liquid-based, water-based or organic solvent-based liquid;
the catholyte, comprising a solvent-I, a cathode active material, a supporting solute and optionally, a functional additive;
the anolyte, comprising a solvent-II, an anode active material, the supporting solute, and optionally, a functional additive;
wherein the ion exchange liquid membrane separates the catholyte and the anolyte by independently stratifying with the catholyte and the anolyte, dissolves the supporting solute, but does not dissolve the cathode and anode active materials.

There is no special requirement for the solvent-I and solvent-II, as long as they are suitable for use in flow batteries and enable the prepared catholyte and anolyte satisfying the above requirements. Appropriate solvent-I and solvent-II can be selected through routine experiments according to the above-mentioned relationship requirements for the catholyte, the anolyte and the ion exchange liquid membrane in the flow battery of the present invention.

The solvent-I and solvent-II may be the same or different, preferably the same, and are each independently selected from water, a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound, and a mixture thereof, but the present invention is not limited thereto.

In particular, the solvent-I and the solvent-II are each independently selected from water, trimethyl phosphate, triethyl phosphate, triphenyl phosphate, tris(2,2,2-trifluoroethyl) phosphate, N,N-dimethylformamide, N,N-dimethylacetamide, N-methylpropionamide, trifluoroacetamide, N-methyltrifluoroacetamide, N,N-dimethyltrifluoroacetamide, hexamethyldisiloxane, hexa(trifluoropropyl)disiloxane, trifluoropropylmethylcyclotrisiloxane, aminopropyl pentamethyl disiloxane, hydroxymethyl pentamethyl disiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, etc., and a mixture thereof.

In the present specification,

The phosphate compound is an organic ester derivative of phosphoric acid, which has a moiety of

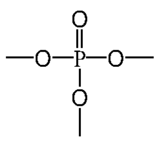

in the molecular structure, and can generally be represented as $P(=O)(OR)_3$, wherein each R is independently selected from C1-C6 alkyl, C1-C6 haloalkyl, and C6-C12 aryl, but the present invention is not limited thereto; for example, the phosphate compound can be trimethyl phosphate, triethyl phosphate, triphenyl phosphate, tris(2,2,2-trifluoroethyl) phosphate, or the like;
The amide compound is an organic compound obtained by replacing the hydroxyl group in the carboxylic acid by an amino or amine group, which has a moiety of

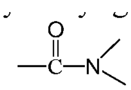

in the molecular structure, and can generally be represented as $R_1C(=O)NR_2R_3$, wherein $R_1$ is selected from H, C1-C6 alkyl, C1-C6 haloalkyl, C6-C12 aryl, and amino ($-NH_2$), and $R_2$ and $R_3$ are each independently selected from H, C1-C6 alkyl, and C1-C6 haloalkyl, but the present invention is not limited thereto; for example, the amide compound can be N,N-dimethylformamide, N,N-dimethylacetamide, N-methylpropionamide, trifluoroacetamide, N-methyltrifluoroacetamide, N,N-dimethyltrifluoroacetamide, or the like;

The siloxane compound is an organic compound containing a silicon-oxygen bond in its skeleton, which has a moiety of

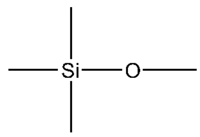

in the molecular structure, and can generally be represented as $R_1OSiR_2R_3R_4$, wherein $R_1$, $R_2$, $R_3$, and $R_4$ can be independently selected from C1-C6 alkyl, C1-C6 alkoxy, C1-C6 haloalkyl, C1-C6 haloalkoxy, and a silyl group, but the present invention is not limited thereto; wherein, the silyl group is a group connected through a silicon atom, which can generally be represented as $R_1R_2R_3Si$-, where $R_1$, $R_2$, and $R_3$ can be independently selected from C1-C6 alkyl, C1-C6 alkoxy, C1-C6 alkyl substituted by halogen, hydroxyl or amino (such as trifluoromethyl, trifluoropropyl, hydroxymethyl, aminomethyl, aminopropyl, etc.), C1-C6 alkoxy substituted by halogen, hydroxyl or amino, but the present invention is not limited thereto. For example, the siloxane compound may be hexamethyldisiloxane, hexa(trifluoropropyl)disiloxane, trifluoropropylmethylcyclotrisiloxane, aminopropylpentamethyldisiloxane, hydroxymethylpentamethyldisiloxane, or the like;

The carbonate compound is an organic ester derivative of carbonic acid, which has a moiety of

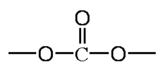

in the molecular structure, and can generally be represented as $R_1OC(=O)OR_2$, wherein Ri and $R_2$ are each independently selected from C1-C6 alkyl, C1-C6 haloalkyl, etc., or $R_1$ and $R_2$ together form a C2-C6 alkylene or C2-C6 haloalkylene, but the present invention is not limited thereto; for example, the carbonate compound can be dimethyl carbonate, ethyl methyl carbonate, diethyl carbonate, ethylene carbonate, propylene carbonate, fluoroethylene carbonate, or the like;

The sulfate compound is an organic ester derivative of sulfuric acid, which has a moiety of

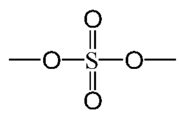

in the molecular structure, and can generally be represented as $R_1OS(=O)_2OR_2$, wherein $R_1$ and $R_2$ are each independently selected from C1-C6 alkyl, and C1-C6 haloalkyl, but the present invention is not limited thereto; for example, the sulfate compound can be dimethyl sulfate, ethyl methyl sulfate, diethyl sulfate, or the like;

The sulfite compound is an organic ester derivative of sulfurous acid, which has a moiety of

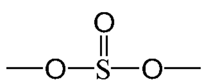

in the molecular structure, and can generally be represented as $R_1OS(=O)OR_2$, wherein $R_1$ and $R_2$ are each independently selected from C1-C6 alkyl, C1-C6 haloalkyl, and the like, or $R_1$ and $R_2$ together form a C2-C6 alkylene or a C2-C6 haloalkylene, but the present invention is not limited thereto; for example, the sulfite compound can be dimethyl sulfite, ethyl methyl sulfite, diethyl sulfite, vinyl sulfate, or the like;

The carboxylate compound is an organic ester derivative of carboxylic acid, which has a moiety of

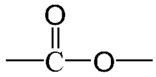

in the molecular structure, and can generally be represented as $R_1C(=O)OR_2$, wherein $R_1$ and $R_2$ are each independently selected from H, C1-C6 alkyl, C1-C6 haloalkyl, and the like, or $R_1$ and $R_2$ together form a C2-C6 alkylene or a C2-C6 haloalkylene, but the present invention is not limited thereto; for example, the carboxylate-based compound can be ethyl acetate, 1,4-butyrolactone, or the like;

The ketone compound is an organic compound in which a carbonyl group is connected to two hydrocarbon groups, which can generally be represented as $R_1C(=O)R_2$, wherein $R_1$ and $R_2$ are each independently selected from C1-C6 alkyl, C1-C6 haloalkyl, and the like, or $R_1$ and $R_2$ together form a C2-C6 alkylene or C2-C6 haloalkylene, but the present invention is not limited thereto; for example, the ketone compound can be 4-methyl-2-pentanone, or the like;

The ether compound is an organic compound with an ether functional group that is formed by connecting two alkyl or aryl with an oxygen atom. The ether compound can generally be represented as $R_1$-O-$R_2$, wherein $R_1$ and $R_2$ are independently selected from C1-C6 alkyl, C1-C6 alkyl substituted by halogen, hydroxyl, C1-C6 alkoxy, and the like, heteroalkyl obtained by replacing one or more carbons with oxygen in the above alkyl, and the like, or $R_1$ and $R_2$ together form a C2-C6 alkylene, a C2-C6 haloalkylene, a heteroalkylene in which one or more carbons in the above alkylene are replaced by oxygen, but the present invention is not limited thereto; for example, the ether compound can be ethylene glycol monomethyl ether, ethylene glycol dimethyl ether, tetraethylene glycol dimethyl ether, tetrahydrofuran, 2-methyltetrahydrofuran, 1,3-dioxolane, or the like;

The sulfone compound is an organic compound that has a sulfonyl group and connects two carbon atoms through a sulfur, which can generally be represented as $R_1S(=O)_2R_2$, wherein $R_1$ and $R_2$ are each independently selected from C1-C6 alkyl, C1-C6 haloalkyl, and the like, or $R_1$ and $R_2$ together form a C2-C6 alkylene or C2-C6 haloalkylene, but the present invention is not limited thereto; for example, the sulfone compound can be dimethyl sulfoxide, or the like;

The alcohol compound is a compound in which a hydrogen atom in the side chain of an aliphatic, alicyclic or aromatic hydrocarbon is replaced by a hydroxyl group, which can generally be represented as RiOH, where $R_1$ is selected from C1-C6 alkyl, C1-C6 haloalkyl, and C3-C10 cycloalkyl, but the present invention is not limited thereto;

The nitrile compound refer to an organic compound with a C=N functional group, which can generally be represented as RiCN, wherein Ri is selected from C1-C6 alkyl, C1-C6 haloalkyl, and C3-C10 cycloalkyl, but the present invention is not limited thereto; for example, the nitrile compound can be acetonitrile, or the like;

The pyridine compound refer to a compound with a pyridine ring as the core, such as pyridine, pyridine substituted by halogen, C1-C6 alkyl, and C1-C6 haloalkyl, but the present invention is not limited thereto; for example, the pyridine compound can be pyridine, methylpyridine, or the like;

The halogenated hydrocarbon compound refer to a compound in which a hydrogen atom in a hydrocarbon molecule is replaced by a halogen atom, which can generally be represented as $R_1X$, where $R_1$ can be an aliphatic or aromatic hydrocarbon group, and X is a halogen, including fluorine, chlorine, bromine, and iodine; for example, the halogenated hydrocarbon compound can be 1,2-dichloroethane, or the like;

The aromatic compound is a compound with an aromatic ring structure that complies with Huckel's rule; for example, the aromatic compound can be nitrobenzene, or the like.

The cathode active material contained in the catholyte has redox activity with two states: oxidized state and reduced state. The two states can be reversibly transformed through electrochemical reaction. The molar ratio of the two states (oxidized state and reduced state) determines the charge state of the flow battery, and the higher the molar ratio is, the higher the charge state of the battery (up to 100%).

There is no particular restriction on the cathode active material contained in the catholyte, as long as it is suitable for use in a flow battery and meets the above-mentioned requirements for its solubility in the catholyte and the ion exchange liquid membrane. For example, the cathode active material can be selected from any cathode active material for flow batteries known in the art, for example, the cathode active material listed in Electrolyte Lifetime in Aqueous Organic Redox Flow Batteries: A Critical Review (Chemical Reviews, 2020, 120(14):6467-6489.), Recent developments in organic redox flow batteries: A critical review (Journal of Power Sources, 2017, 360(1):243-283.), Recent progress in organic redox flow batteries: Active materials, electrolytes and membranes (Journal of Energy Chemistry, 2018:S2095495617311051.) and Molecular redox species for next-generation batteries (Chemical Society Reviews, 2021,50, 5863-5883.), etc. After understanding the inventive concept of the present invention, according to the solubility requirements of the cathode active material in the catholyte and the ion exchange liquid membrane, battery performance, etc., an appropriate cathode active material can be selected according to the ion exchange liquid membrane and the solvent-I selected via routine experiments, or an appropriate ion exchange liquid membrane and solvent-I can be selected

according to the selected cathode active material.

In particular, the cathode active material may be selected from:

(1) potassium ferrocyanide, having a chemical formula of $K_4Fe(CN)_6$, an oxidized state of ferricyanide ion ($[Fe(CN)_6]^{3-}$), and a reduced state of ferrocyanide ion ($[Fe(CN)_6]^{4-}$);

(2) a 2,2,6,6-tetramethylpiperidine oxide (TEMPO) compound, having an oxidized state shown in the following formula 2, usually abbreviated as $R\text{-}C_9NO^+$, and a reduced state shown in the following formula 1, usually abbreviated as $R\text{-}C_9NO$:

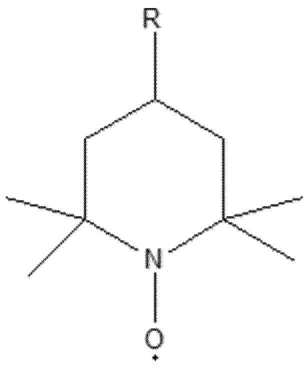

1

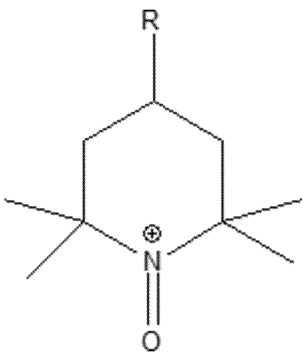

2

wherein, R is selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$);

(3) a ferrocene compound, having an oxidized state shown in the following formula 4, usually abbreviated as $R_1R_2Fe(C_5H_4)_2^+$, and a reduced state shown in the following formula 3, usually abbreviated as $R_1R_2Fe(C_5H_4)_2$:

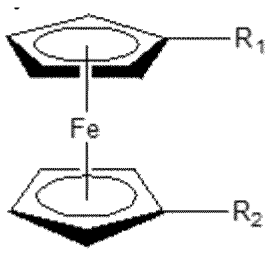

3

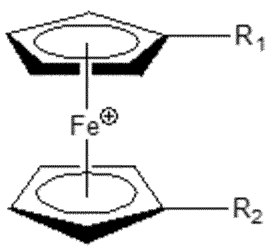

4

wherein, $R_1$ and $R_2$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$);

(4) vanadium (IV) oxide sulfate, having a chemical formula of $VOSO_4$, an oxidized state of pentavalent vanadium ion ($V^{5+}$), and a reduced state of tetravalent vanadium ion ($V^{4+}$);

(5) metal iodide ($MI_n$) or hydrogen iodide (HI), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $I_2$ and the reduced state is $MI_n$ or HI;

(6) 2,5-dimercaptothiadiazole, having a molecular formula of $C_2H_2N_2S_3$, an oxidized state of $(C_2HN_2S_3)_2$, and a reduced state of $C_2H_2N_2S_3$;

(7) a catechol organic compound, having an oxidized state shown in the following formula 6, usually abbreviated as $R_1R_2R_3R_4C_6O_2$, and a reduced state shown in the following formula 5, usually abbreviated as $R_1R_2R_3R_4C_6(OH)_2$:

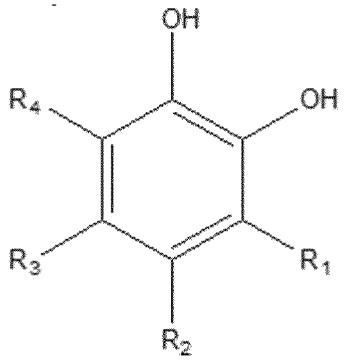

5

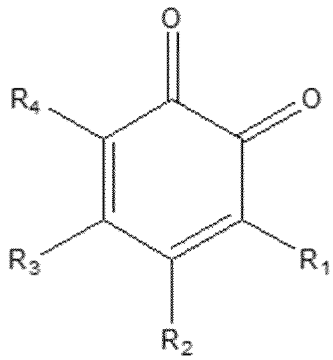

6

wherein, $R_1$, $R_2$, $R_3$ and $R_4$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$),

(8) a divalent iron salt, having a chemical formula of $FeX_n$, wherein X is selected from $Cl^-$, Br, $SO_4^{2-}$, $NO_3^-$, and $ClO_4^-$, n is 1 or 2, and the oxidized state is ferric ion $Fe^{3+}$, the reduced state is ferrous ion $Fe^{2+}$;

(9) metal bromide ($MBr_n$) or hydrogen bromide (HBr), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $Br_2$, and the reduced state is $MBr_n$ or HBr.

The anode active material contained in the anolyte has redox activity with two states: oxidized state and reduced state. The two states can be reversibly transformed through electrochemical reaction. The molar ratio of the two states (oxidized state and reduced state) determines the charge state of the flow battery. The higher the molar ratio of the oxidized state is, the higher the charge state of the battery (up to 100%).

There is no particular restriction on the anode active material contained in the anolyte, as long as it is suitable for use in a flow battery and meets the above-mentioned requirements for its solubility in the anolyte and the ion exchange liquid membrane. For example, the anode active material can be selected from any anode active material for flow batteries known in the art, for example, the anode active material listed in Electrolyte Lifetime in Aqueous Organic Redox Flow Batteries: A Critical Review (Chemical Reviews, 2020, 120(14):6467-6489.), Recent developments in organic redox flow batteries: A critical review (Journal of Power Sources, 2017, 360(1):243-283.), Recent progress in organic redox flow

batteries: Active materials, electrolytes and membranes (Journal of Energy Chemistry, 2018:S2095495617311051.) and Molecular redox species for next-generation batteries (Chemical Society Reviews, 2021,50, 5863-5883.), etc. After understanding the inventive concept of the present invention, according to the solubility requirements of the anode active material in the anolyte and the ion exchange liquid membrane, battery performance, etc., an appropriate anode active material can be selected according to the ion exchange liquid membrane and the solvent-II selected via routine experiments, or an appropriate ion exchange liquid membrane and solvent-II can be selected according to the selected anode active material.

In particular, the anode active material may be selected from:

(1) a divalent zinc salt, having a chemical formula of $ZnX_n$, wherein X is selected from $Cl^-$, Br, $SO_4^{2-}$, $NO_3^-$, $CH_3COO^-$, $TFSI^-$, and OTf, n is 1 to 2, the oxidized state is zinc ion ($Zn^{2+}$), and the reduced state is metallic zinc;

(2) methyl viologen (MV), having a molecular formula of $C_{12}H_{14}Cl_2N_2$, an oxidized state of $MV^{2+}$, and a reduced state of $MV^+$;

(3) an acetylacetone metal salt, having a chemical formula of $M(acac)_n$, an oxidized state of metal ion ($M^{n+}$), and a reduced state of metal element M or a lower-valent acetylacetone metal salt ($M^{m+}$), wherein M is selected from Li, Fe, Al, Ni, Be, Ga, In, V, Cr, Co, Mn, Cu, Pt, Ru, Ti, Cd, and Mo, n is an integer from 1 to 4 , m is an integer from 1 to 3, and m<n;

(4) a p-benzoquinone compound, having an oxidized state shown in the following formula 7, usually abbreviated as $R_1R_2R_3R_4C_6O_2$, and a reduced state shown in the following formula 8, usually abbreviated as $R_1R_2R_3R_4C_6OH_2$:

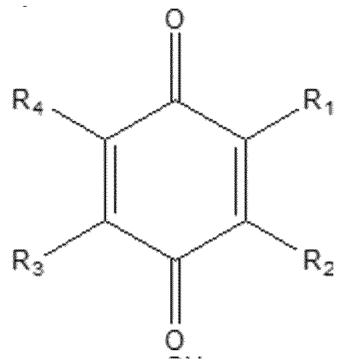

7

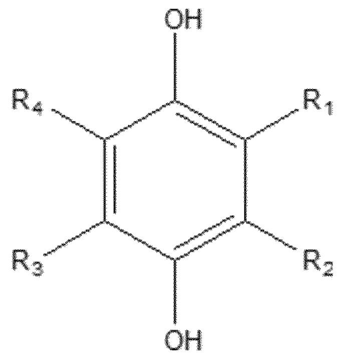

8

wherein, $R_1$, $R_2$, $R_3$ and $R_4$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, sulfo ($-SO_3H$);

(5) an anthraquinone compound, having an oxidized state shown in the following formula 9, usually abbreviated as $R_1R_2R_3R_4R_5R_6R_7R_8C_{14}O_2$, and a reduced state shown in the following formula 10, usually abbreviated as $R_1R_2R_3R_4R_5R_6R_7R_8C_{14}(OH)_2$:

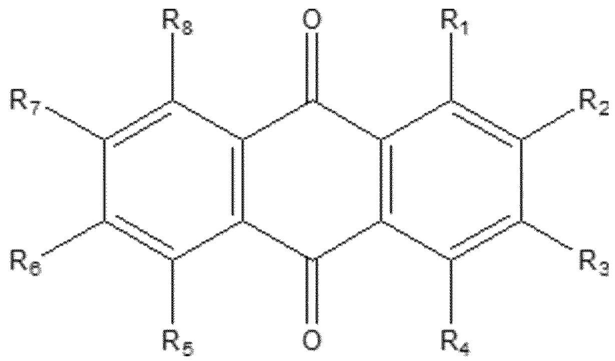

9

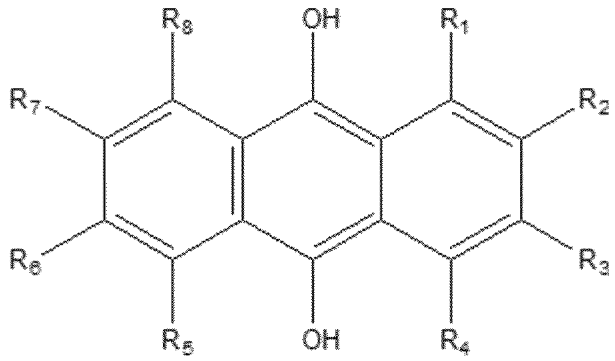

10

wherein, $R_1$, $R_2$, $R_3$, $R_4$, $R_5$, $R_6$, $R_7$, and $R_8$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$);

(5) a phenazine compound, having an oxidized state shown in the following formula 11, usually abbreviated as $R_1R_2R_3R_4R_5R_6R_7R_8C_{12}N_2$, and a reduced state shown in the following formula 12, usually abbreviated as $R_1R_2R_3R_4R_5R_6R_7R_8C_{12}N_2H_2$:

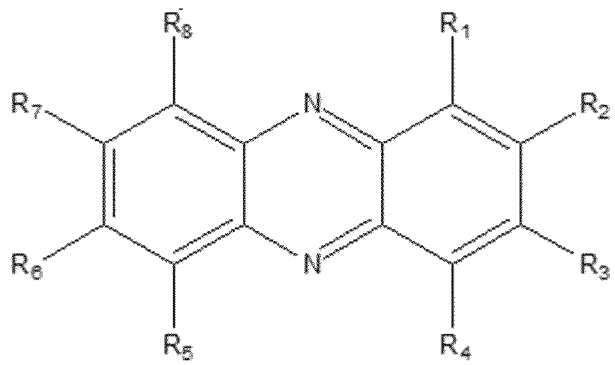

11

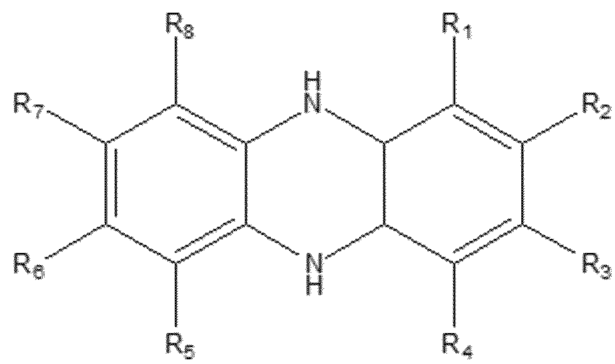

12

wherein, $R_1$, $R_2$, $R_3$, $R_4$, $R_5$, $R_6$, $R_7$, and $R_8$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$);

(7) vanadium sulfate (III), having a chemical formula of $V_2(SO_4)_3$, an oxidized state of trivalent vanadium ion ($V^{3+}$), and a reduced state of divalent vanadium ion ($V^{2+}$);

(8) camphorquinone, having a molecular formula of $C_{10}H_{14}O_2$, an oxidized state of $C_{10}H_{14}O_2$, and a reduced state of $C_{10}H_{14}O_2^-$;

(9) N-methylphthalimide, having a molecular formula of $C_9H_7NO_2$, an oxidized state of $C_9H_7NO_2$, and a reduced state of $C_9H_7NO_2^-$;

(10) a fluorenone compound, having an oxidized state shown in the following formula 13, usually abbreviated as $R_1R_2R_3R_4R_5R_6R_7R_8C_{13}O$, and a reduced state shown in the following formula 14, usually abbreviated as $R_1R_2R_3R_4R_5R_6R_7R_8C_{13}OH$:

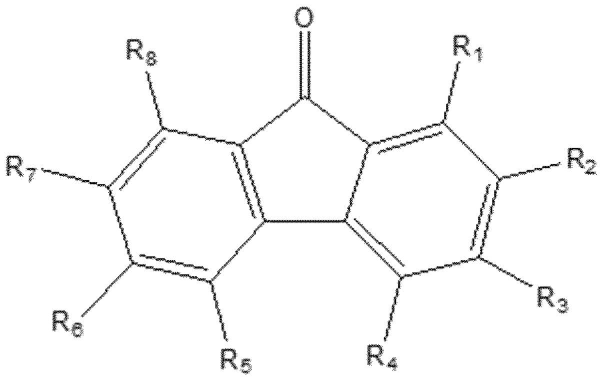

13

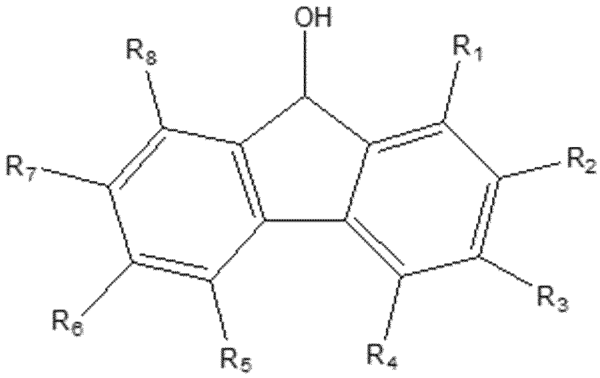

14

wherein, $R_1$, $R_2$, $R_3$, $R_4$, $R_5$, $R_6$, $R_7$, and $R_8$ are each independently selected from hydrogen, hydroxyl, carboxyl, halogen, C1 to C20 alkyl, C1 to C20 alkoxy, amino ($-NH_2$), C1 to C20 alkylamino, carboxyl C1 to C20 alkylamino, C1 to C20 amide group, C6 to C20 aryl, and sulfo ($-SO_3H$);

(11) a trivalent chromium salt, having a chemical formula of $CrX_n$, wherein X is selected from $Cl^-$, Br, $SO_4^{2-}$, $NO_3^-$, and $ClO_4^-$, n is 1.5 or 3, and the oxidized state is trivalent chromium ion ($Cr^{3+}$), and the reduced state is divalent chromium ion ($Cr^{2+}$).

In the above definition,

Halogen includes fluorine (F), chlorine (Cl), bromine (Br) and iodine (I);

C1 to C20 alkyl refers to a straight or branched alkyl with 1 to 20 carbon atoms, including methyl, ethyl, n-propyl, isopropyl, n-butyl, isobutyl, tert-butyl, but the present invention is not limited thereto.

C1 to C20 alkoxy refers to a group in which the hydrogen on the hydroxyl group (-OH) is replaced by a straight or branched alkyl group of 1 to 20 carbon atoms, for example, it can be a methoxy ($-OCH_3$) , ethoxy ($-OCH_2CH_3$), but the present invention is not limited thereto.

C1 to C20 alkylamino refers to a group in which one or two hydrogens on amino ($-NH_2$) are replaced with the

above-mentioned C1 to C20 alkyl. For example, it can be methylamino ($-NHCH_3$), dimethylamino ($-N(CH_3)_2$), ethylamino ($-NHCH_2CH_3$), diethylamino ($-N(CH_2CH_3)_2$), but the present invention is not limited thereto.

Carboxyl C1 to C20 alkylamino refers to a group in which one or two hydrogen atoms on amino ($-NH_2$) are replaced with a carboxyl C1 to C20 alkyl. For example, it can be acetic acid amino ($-NHCH_2COOH$), propionic acid amino ($-NHCH_2CH_2COOH$), but the present invention is not limited thereto.

C1 to C20 amide group refers to a group in which the hydroxyl (-OH) on the carboxyl (-COOH) is replaced by amino ($-NH_2$) or a C1 to C19 alkylamino. For example, it can be aminocarboonyl ($-CONH_2$), methylaminocarbonyl ($-CONHCH_3$), but the present invention is not limited thereto.

C6 to C20 aryl refers to an aromatic carbocyclic hydrocarbon group having 1 to 20 carbon atoms, such as phenyl, tolyl, xylyl, ethylphenyl, naphthyl, etc.

The substituents that may exist in the above-mentioned active materials may be selected by comprehensively considering the requirement for the properties of the active materials such as solubility, stability, electrochemical redox reversibility, etc. In particular, when there is no need to modify the active material molecule by substitution, all substituents are considered to be hydrogen atoms.

The supporting solute is soluble in each of the ion exchange liquid membrane and the catholyte and anolyte, and is used to balance the charge states of the cathode and anode active materials during the charge and discharge process through the ion conduction, so as to ensure the overall electrical neutrality.

The expression that the supporting solute is soluble in each of the ion exchange liquid membrane, the catholyte and the anolyte means that the soluble amounts of the supporting solute in the ion exchange liquid membrane, the catholyte and the anolyte are independently not less than 0.1 mol/L, preferably 0.2 to 5 mol/L. The specific concentration is determined by the ionic conductivity in the battery system. The concentration of the supporting solute affects the conductivity of the electrolyte, which in turn affects the rate capability of the battery. If the concentration is too low, the conductivity is low and the rate capability is poor. If the concentration is too high, the viscosity is high and the conductivity becomes smaller. Therefore, under the guidance of the technical disclosure of the present application, the appropriate concentration of the supporting solute can be determined through simple experiments according to the specific required conductivity.

The cation of the supporting solute is not particularly limited as long as it is suitable for flow batteries. For example, it can be a monovalent or multivalent cation that is inert in electrochemical redox reaction, such as one or more selected from $H^+$, $NH_4^+$, $Li^+$, $Na^+$, $K^+$, $Zn^{2+}$, $Ca^{2+}$, $Mg^{2+}$, $EMIM^+$, $PMIM^+$, and $BMIM^+$, but the present invention is not limited thereto. The expression of being inert in electrochemical redox reaction means that the selected cation will not undergo oxidative or reductive reaction under the working condition (temperature, voltage, pH value, selection of cathode and anode active materials, etc.) of the flow battery.

The anion of the supporting solute is not particularly limited as long as it is suitable for flow batteries. For example, it can be a monovalent or multivalent anion that is inert in electrochemical redox reaction, such as one or more selected from $OH^-$, $F^-$, $Cl^-$, Br, $I^-$, $PF_6^-$, $BF_4^-$, $TFSI^-$, $NO_3^-$, $HCO_3^-$, $ClO_4^-$, $SO_4^{2-}$, $PO_4^{3-}$, and $OTf^-$, but the present invention is not limited thereto. The expression of being inert in electrochemical redox reaction means that, the selected anion will not undergo oxidative or reductive reaction under the working condition (temperature, voltage, pH value, selection of cathode and anode active materials, etc.) of the flow battery.

In particular, the supporting solute may be one or more selected from KCl, $NH_4Cl$, LiCl, NaCl, $CaCl_2$, $MgCl_2$, EMIMCl, PMIMCl, BMIMCl, $LiNO_3$, LiBr, EMIMBr, PMIMBr, BMIMBr, KI, EMIMI, PMIMI, BMIMI, $LiPF_6$, $NaPF_6$, $LiBF_4$, $NaBF_4$, LiTFSI, $LiClO_4$, and LiOTf. The type of the selected supporting solute can be determined according to the type of the selected ion exchange liquid membrane and the properties of the solvents for the catholyte and anolyte through simple dissolution experiments.

The catholyte and anolyte can further comprise an optional functional additive, such as a salting-out functional additive added to assist or achieve stratification of the catholyte and anolyte with the ion exchange liquid membrane; a pH adjustment functional additive added to adjust pH; and an other functional additive added to expand the electrochemical stability window, to adjust the density of the electrolyte, to adjust the solubility of active substances, or the like, but the present invention is not limited thereto.

The salting out functional additive is applied for an organic solvent-based ion exchange liquid membrane or a water-based ion exchange liquid membrane. It is characterized in that the salting out functional additive is only soluble in water but not in the selected organic solvent (that is, when the ion exchange liquid membrane is organic solvent-based, the salting out functional additive is only soluble in the aqueous catholyte and anolyte; and when the ion exchange liquid membrane is water-based, the salting out functional additive is only soluble in the ion exchange liquid membrane but not in organic catholyte and anolyte), and functions to achieve the stratification of water and water-soluble organic solvents, or to assist in strengthening the stratification effect of water and non-water-soluble organic solvents. For details on the mechanism of salting-out function, please refer to Critical Appraisal of Salting-Out and Its Implications for Chemical and Biological Sciences (Chemical Reviews, 2005, 105(1):1-10.).

The cation of the salting-out functional additive is not particularly limited as long as it is suitable for flow batteries.

For example, it can be a monovalent or multivalent cation that is inert in electrochemical redox reaction, including one or more selected from $Li^+$, $K^+$, $NH_4^+$, $Na^+$, $Mg^{2+}$, $Ca^{2+}$, $Ba^{2+}$, and $Ni^{2+}$, but the present invention is not limited thereto. The expression of being inert in electrochemical redox reaction means that, the selected cation will not undergo oxidative or reductive reaction under the working conditions (temperature, voltage, pH value, selection of cathode and anode active materials, etc.) of the flow battery.

The anion of the salting-out functional additive is not particularly limited as long as it is suitable for flow batteries. For example, it can be a monovalent or multivalent anion that is inert in electrochemical redox reaction, including one or more selected from $Cl^-$, Br, $I^-$, $ClO_4^-$, $SCN^-$, $CH_3COO^-$, $SO_4^{2-}$, and $PO_4^{3-}$, but the present invention is not limited thereto. The expression of being inert in electrochemical redox reaction means that, the selected anion will not undergo oxidative or reductive reaction under the working conditions (temperature, voltage, pH value, selection of cathode and anode active materials, etc.) of the flow battery.

In particular, the salting out functional additive can be one or more selected from LiCl, LiBr, $LiClO_4$, $CH_3COOLi$, $Li_2SO_4$, KCl, KBr, $K_2SO_4$, $K_3PO_4$, $NH_4Cl$, $NH_4Br$, $NH_4I$, $NH_4SCN$, $(NH_4)_2SO_4$, NaCl, NaBr, $Na_2SO_4$, $Na_3PO_4$, $MgCl_2$, $MgBr_2$, $MgSO_4$, CaCl, $CaBr_2$, $CaSO_4$, $BaSO_4$, $NiSO_4$. The type of the selected salting-out function additive can be determined through simple solvent stratification experiments or by consulting the literature according to the type of the selected ion exchange liquid membrane and the properties of the solvents for the catholyte and anolyte. For details on the selection range and selection method of the salting out functional additive, please refer to Salting out of proteins using ammonium sulfate precipitation (Methods in enzymology, 2014, 541(541):85-94.), Mechanism of protein salting in and salting out by divalent cation salts: balance between hydration and salt binding (Biochemistry, 1984, 23(25):5912-5923.) and Salting-In and Salting-Out of Water-Soluble Polymers in Aqueous Salt Solutions (Journal of Physical Chemistry B, 2012, 116(17):5234-5241.).

The pH adjustment function additive is applied when the solvent of the catholyte and anolyte is water, and functions to adjust the pH of the catholyte and anolyte. The selected pH adjustment function additive is not particularly limited, as long as it is suitable for flow batteries and inert in electrochemical redox reaction. For example, the pH adjustment function additive can be selected from the common acids or bases, such as HCl, $H_2SO_4$, $HNO_3$, $H_3PO_4$, $H_3BO_3$, $CH_3COOH$, NaOH, KOH, $NaHCO_3$, $NaHPO_4$, $NaH_2PO_4$ and the like. The expression of being inert in electrochemical redox reaction means that, the selected pH adjustment function additive will not undergo oxidative or reductive reaction under the working conditions (temperature, voltage, pH value, selection of cathode and anode active materials, etc.) of the flow battery.

Other functional additives can be flexibly selected according to the functional requirements of the flow battery system used by those skilled in the art, as long as they are suitable for flow batteries and inert in electrochemical redox reaction. For example, in order to expand the electrochemical stability window of aqueous electrolytes, an additive or a co-solvent such as triethyl phosphate, acetonitrile, urea, or methyl urea can be added to the water to broaden the electrochemical stability window; for example, in order to adjust the density of aqueous electrolytes, water-soluble organic solvents with different densities can be introduced to increase (such as propylene carbonate) or decrease (such as ethyl acetate) the density of the electrolyte; for example, in order to increase the solubility of the organic active substance in water, ethanol as a latent solvent or ethylene diamine as a co-solvent can be added to increase the solubility, but the present invention is not limited thereto. The expression of being inert in electrochemical redox reaction means that, the selected other functional additives will not undergo oxidative or reductive reaction under the working conditions (temperature, voltage, pH value, selection of cathode and anode active materials, etc.) of the flow battery.

The present invention provides three types of ion exchange liquid membranes which are ionic liquid-, organic solvent-, and water-based, respectively, so as to adapt to various types of catholyte and anolyte ranging from aqueous systems to organic systems. Certain embodiments of single-cell flow batteries constructed based on the above three types of ion exchange liquid membranes will be described in detail in the following description.

**[Ionic liquid-based ion exchange liquid membrane]**

In one aspect, the present invention provides a flow battery including:

a catholyte, comprising a solvent-I, a cathode active material, a supporting solute and optionally, a functional additive;
an anolyte, comprising a solvent-II, an anode active material, the supporting solute and optionally, a functional additive;
an ion exchange liquid membrane, which is an ionic liquid-based liquid, separates the catholyte and the anolyte by independently stratifying with the catholyte and the anolyte, dissolves the supporting solute, but does not dissolve the cathode and anode active materials.

The descriptions with respect to the catholyte and the anolyte, the solvent-I and the solvent-II, the cathode and anode active materials, the supporting solute, the functional additive, and the ionic liquid are the same as the above and

will not be repeated here.

**[Organic solvent-based ion exchange liquid membrane]**

In one aspect, the present invention provides a flow battery including:

a catholyte, comprising water, a cathode active material, a supporting solute and optionally, a functional additive;
an anolyte, comprising water, an anode active material, the supporting solute and optionally a functional additive;
an ion exchange liquid membrane, which is an organic solvent-based liquid, separates the catholyte and the anolyte by independently stratifying with the catholyte and the anolyte, dissolves the supporting solute, but does not dissolve the cathode and anode active materials, thereby providing a pathway for the transport of the supporting solute ions between the catholyte and the anolyte.

There is no particular restriction on the organic solvent suitable for use in the ion exchange liquid membrane as long as it meets the above requirement and is suitable for use in batteries. In particular, the organic solvent suitable for use in the ion exchange liquid membrane can be selected from: a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound and a mixture thereof, but the present invention is not limited thereto.

In particular, the organic solvent may be selected from trimethyl phosphate, triethyl phosphate, triphenyl phosphate, tris(2,2,2-trifluoroethyl) phosphate, N,N-dimethylformamide, N,N-dimethylacetamide, N-methylpropionamide, trifluoroacetamide, N-methyltrifluoroacetamide, N,N-dimethyltrifluoroacetamide, hexamethyldisiloxane, hexa(trifluoropropyl)disiloxane, trifluoropropylmethylcyclotrisiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, etc., and a mixture thereof.

There is no particular restriction on the cathode active material contained in the catholyte, as long as it is suitable for use in an aqueous flow battery. For example, the cathode active material can be any cathode active material for aqueous flow batteries known in the art, for example, a cathode active material listed in Electrolyte Lifetime in Aqueous Organic Redox Flow Batteries: A Critical Review (Chemical Reviews, 2020, 120(14):6467-6489.), and Molecular redox species for next-generation batteries (Chemical Society Reviews, 2021,50, 5863-5883.), etc. In particular, the cathode active material may be selected from:

(1) the above-mentioned potassium ferrocyanide;
(2) a water-soluble 2,2,6,6-tetramethylpiperidine oxide (TEMPO) compound, which has the same structure as the above-mentioned TEMPO compound, except that R is selected from hydroxyl, carboxyl, amino, carboxyl C1 to C20 alkylamino, C1 to C20 amide group, and sulfo to ensure the water solubility of the compound;
(3) a water-soluble ferrocene compound, which has the same structure and substituent definitions as the above-mentioned ferrocene compound, except that at least one of $R_1$ and $R_2$ is selected from hydroxyl, carboxyl, amino, carboxyl C1 to C20 alkylamino, C1 to C20 amide group, and sulfo to ensure the water solubility of the compound;
(4) the above-mentioned vanadium(IV) oxide sulfate;
(5) the above-mentioned metal iodide ($MI_n$) or hydrogen iodide (HI);
(6) a water-soluble catechol organic compound, which has the same structure and substituent definitions as the above-mentioned catechol compound, except that at least one of $R_1$, $R_2$, $R_3$, and $R_4$ is selected from hydroxyl, carboxyl, amino, carboxyl C1 to C20 alkylamino, C1 to C20 amide group, and sulfo to ensure the water solubility of the compound;
(7) the above-mentioned divalent iron salt;
(8) the above-mentioned metal bromide ($MBr_n$) or hydrogen bromide (HBr).

There is no particular restriction on the anode active material contained in the anolyte, as long as it is suitable for use in an aqueous flow battery. For example, the anode active material can be any anode active material for aqueous flow batteries known in the art, for example, an anode active material listed in Electrolyte Lifetime in Aqueous Organic Redox Flow Batteries: A Critical Review (Chemical Reviews, 2020), and Molecular redox species for next-generation batteries (Chemical Society Reviews, 2021), etc.

In particular, the anode active material may be selected from:

(1) a water-soluble divalent zinc salt, having a chemical formula of $ZnX_n$, wherein X is selected from $Cl^-$, $Br^-$, $SO_4^{2-}$, $NO_3^-$, and $CH_3COO^-$, and n is 1 to 2;
(2) the above-mentioned methyl viologen;
(3) a water-soluble p-benzoquinone compound, which has the same structure and substituent definitions as the above-mentioned p-benzoquinone compound, except that at least one of $R_1$, $R_2$, $R_3$, and $R_4$ is selected from hydroxyl, carboxyl, amino, carboxyl C1 to C20 alkylamino, C 1 to C20 amide group, and sulfo to ensure the water solubility of the compound;
(4) a water-soluble anthraquinone compound, which has the same structure and substituent definitions as the above-mentioned anthraquinone compound, except that at least one of $R_1$, $R_2$, $R_3$, $R_4$, $R_5$, $R_6$, $R_7$, and Rs is selected from hydroxyl, carboxyl, amino, carboxyl C1 to C20 alkylamino, C 1 to C20 amide group, and sulfo to ensure the water solubility of the compound;
(5) a water-soluble phenazine compound, which has the same structure and substituent definitions as the above-mentioned phenazine compound, except that at least one of $R_1$, $R_2$, $R_3$, $R_4$, $R_5$, $R_6$, $R_7$, and $R_8$ is selected from hydroxyl, carboxyl, amino, carboxyl C1 to C20 alkylamino, C 1 to C20 amide group, and sulfo to ensure the water solubility of the compound;
(6) the above-mentioned vanadium sulfate (III);
(7) a water-soluble fluorenone compound, which has the same structure and substituent definitions as the above-mentioned fluorenone compound, except that at least one of $R_1$, $R_2$, $R_3$, $R_4$, $R_5$, $R_6$, $R_7$, and $R_8$ is selected from hydroxyl, carboxyl, amino, carboxyl C1 to C20 alkylamino, C 1 to C20 amide group, and sulfo to ensure the water solubility of the compound;
(8) the above-mentioned trivalent chromium salt.

The selected supporting solute should also be suitable for aqueous flow batteries, that is, it can be dissolved in both of water and the organic solvent selected for the ion exchange liquid membrane. For example, if dimethyl carbonate is selected for the ion exchange liquid membrane, then $LiClO_4$, which is soluble in both of water and dimethyl carbonate, can be selected as the supporting solute, but NaCl, which is only soluble in water but not in dimethyl carbonate, cannot be selected. Apart from this, the description of the supporting solute is the same as the above and will not be repeated here.
The descriptions with respect to the functional additives are the same as the above and will not be repeated here.
Here, an aqueous flow battery refers to a flow battery in which both the catholyte and the anolyte use water as the solvent.

**[Water-based ion exchange liquid membrane based on water]**

In one aspect, the present invention provides a flow battery including:

a catholyte, comprising an organic solvent-I, a cathode active material, a supporting solute and optionally, a functional additive;
an anolyte, comprising an organic solvent-II, an anode active material, the supporting solute and optional, a functional additive;
an ion exchange liquid membrane, which is an water-based liquid, separates the catholyte and the anolyte by independently stratifying with the catholyte and the anolyte, dissolves the supporting solute, but does not dissolve the cathode and anode active materials.

Herein, the organic solvent-I and the organic solvent-II may be independently selected from a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound and a mixture thereof, but the present invention is not limited thereto.
Particularly, the organic solvent-I and organic solvent-II may be independently selected from:
trimethyl phosphate, triethyl phosphate, triphenyl phosphate, tris(2,2,2-trifluoroethyl) phosphate, N,N-dimethylformamide, N,N-dimethylacetamide, N-methylpropionamide, trifluoroacetamide, N-methyltrifluoroacetamide, N,N-dimethyltrifluoroacetamide, hexamethyldisiloxane, hexa(trifluoropropyl)disiloxane, trifluoropropylmethylcyclotrisiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, etc., and a mixture thereof.
There is no particular restriction on the cathode active material contained in the catholyte, as long as it is suitable for use in an organic flow battery. For example, the cathode active material can be any cathode active material for organic

flow batteries known in the art, for example, a cathode active material listed in Recent developments in organic redox flow batteries: A critical review (Journal of Power Sources, 2017, 360(1):243-283.), Recent progress in organic redox flow batteries: Active materials, electrolytes and membranes (Journal of Energy Chemistry, 2018:S2095495617311051.) and Molecular redox species for next-generation batteries (Chemical Society Reviews, 2021,50, 5863-5883.), etc.

In particular, the cathode active material may be selected from:

(1) a water-insoluble 2,2,6,6-tetramethylpiperidine oxide (TEMPO) compound, which has the same structure as the above-mentioned TEMPO compound, except that R is selected from hydrogen, halogen, C1 to C20 alkyl, C1 to C20 alkoxy, C1 to C20 alkylamino, C6 to C20 aryl;
(2) a water-insoluble ferrocene compound, which has the same structure and substituent definitions as the above-mentioned ferrocene compound, except that $R_1$ and $R_2$ are each independently selected from hydrogen, halogen, C1 to C20 alkyl, C1 to C20 alkoxy, C1 to C20 alkylamino, and C6 to C20 aryl;
(3) the above-mentioned 2,5-dimercaptothiadiazole;
(4) a water-insoluble catechol compound, which has the same structure as the above-mentioned catechol compound, except that $R_1$, $R_2$, $R_3$, and $R_4$ are each independently selected from hydrogen, halogen, C1 to C20 alkyl, C1 to C20 alkoxy, C1 to C20 alkylamino, and C6 to C20 aryl.

There is no particular restriction on the anode active material contained in the anolyte, as long as it is suitable for use in an organic flow battery. For example, the anode active material can be any anode active material for organic flow batteries known in the art, for example, anode active material listed in Recent developments in organic redox flow batteries: A critical review (Journal of Power Sources, 2017, 360(1):243-283.), Recent progress in organic redox flow batteries: Active materials, electrolytes and membranes (Journal of Energy Chemistry, 2018:S2095495617311051.) and Molecular redox species for next-generation batteries (Chemical Society Reviews, 2021,50, 5863-5883.), etc.

In particular, the anode active material may be selected from:

(1) the above-mentioned acetylacetone metal salt;
(2) a water-insoluble divalent zinc salt, having a chemical formula of $ZnX_2$, wherein X is selected from $TFSI^-$, and $OTf^-$, the oxidized state is zinc ion ($Zn^{2+}$), and the reduced state is metallic zinc;
(3) a water-insoluble p-benzoquinone compound, which has the same structure as the above-mentioned p-benzoquinone compound, except that $R_1$, $R_2$, $R_3$, and $R_4$ are each independently selected from hydrogen, halogen, C1 to C20 alkyl, C1 to C20 alkoxy, C1 to C20 alkylamino, and C6 to C20 aryl;
(4) a water-insoluble anthraquinone compound, which has the same structure as the above-mentioned anthraquinone compound, except that $R_1$, $R_2$, $R_3$, $R_4$, $R_5$, $R_6$, $R_7$ and $R_8$ are each independently selected from hydrogen, halogen, C1 to C20 alkyl, C1 to C20 alkoxy, C1 to C20 alkylamino, and C6 to C20 aryl;
(5) the above-mentioned camphorquinone;
(6) the above-mentioned N-methylphthalimide;
(7) a water-insoluble phenazine compound, which has the same structure as the above-mentioned phenazine compound, except that $R_1$, $R_2$, $R_3$, $R_4$, $R_5$, $R_6$, $R_7$ and $R_8$ are each independently selected from hydrogen, halogen, C1 to C20 alkyl, C1 to C20 alkoxy, C1 to C20 alkylamino, and C6 to C20 aryl;
(8) a water-insoluble fluorenone compound, which have the same structure as the above-mentioned fluorenone compound, except that $R_1$, $R_2$, $R_3$, $R_4$, $R_5$, $R_6$, $R_7$ and $R_8$ are each independently selected from hydrogen, halogen, C1 to C20 alkyl, C1 to C20 alkoxy, C1 to C20 alkylamino, and C6 to C20 aryl.

The selected supporting solute should also be suitable for organic flow batteries, that is, it can be dissolved in both of the selected organic solvent for the catholyte and anolyte and water as the solvent for the ion exchange liquid membrane. For example, if dimethyl carbonate is selected as the solvent for the both catholyte and anolyte, then $LiClO_4$, which is soluble in both water and dimethyl carbonate, can be selected as the supporting solute, but NaCl, which is only soluble in water but not in dimethyl carbonate, cannot be selected. Apart from this, the description with respect to the supporting solute is the same as the above and will not be repeated here.

The descriptions with respect to the functional additives are the same as the above and will not be repeated here.

Here, an organic flow battery refers to a flow battery in which the solvents of both the catholyte and anolyte are organic solvents.

The ion exchange liquid membrane flow battery according to the present invention also includes other devices required for conventional flow battery, such as, without limitation, electrolyte tanks, anode current collectors, cathode current collectors, electrolyte circulation pumps and pipelines, etc.

The electrolyte storage tanks may include a catholyte tank and an anolyte tank. There is no particular limitation on the electrolyte tank, and any storage tank suitable for storing electrolyte can be used.

The anode current collector may be any anode current collector that can be used in flow battery in the art

without particular limitation. For example, the anode current collector may be a flat or porous metal electrode with good conductivity; a carbon, porous carbon paper or porous carbon felt (graphite felt) electrode with good conductivity and chemical inertness, or the like.

The cathode current collector may be any cathode current collector that can be used in flow battery in the art without particular limitation. For example, the cathode current collector may be a flat or porous metal electrode with good conductivity; a carbon, porous carbon paper or porous carbon felt (graphite felt) electrode with good conductivity and chemical inertness, or the like.

The electrolyte circulation pumps include a circulation pump for circulating the catholyte and a circulation pump for circulating the anolyte. The electrolyte in the catholyte and anolyte tanks passes through the pipeline, flows through the cathode reaction chamber and anode reaction chamber respectively under the action of the circulation pump, and then flows back to the electrolyte tank through the other end of the pipeline.

According to the difference in density between the ion exchange liquid membrane and the catholyte and anolyte, the flow battery of the present invention can have different single-cell structures. Therefore, there is no particular limitation on the single cell structure of the flow battery of the present invention, as long as the flow battery can realize its function.

Fig. 1 schematically shows a schematic design of a single-cell structure that can be used in the flow battery of the present invention, but the single-cell structure of the flow battery of the present invention is not limited thereto. As shown in Fig. 1, the single-cell structure of the flow battery of the present invention may include a square tank, in which an intermediate bulkhead 1 is placed at a certain height from the bottom to divide the square tank into cathode and anode reaction chambers 2 and 3 located at the upper part of the square tank, and the ion exchange liquid membrane 4 located at the bottom of the square tank; storage tanks 5 and 6 used to store the catholyte and the anolyte; pipes 7 or 8 used to connect the storage tanks 5 or 6 with the cathode or anode chamber 2 or 3 respectively; peristaltic pumps 9 and 10 provided on pipelines 7 and 8 for transporting electrolytes; current collectors (not shown) provided in the cathode and anode reaction chambers 2 and 3, etc.

The single-cell structure of the flow battery can also be U-shaped, in which the ion exchange liquid membrane is located in the middle to separate the catholyte and anolyte. A suitable single-cell structure of the flow battery can be designed according to the density difference between the catholyte and the anolyte and the ion exchange liquid membrane as well as suitable process requirements. Therefore, the present invention is not limited to the above-described structure.

The invention also relates to a battery stack comprising at least one ion exchange liquid membrane flow battery according to the invention.

In an embodiment, the battery stack may be composed of two or more single cells of the ion exchange liquid membrane flow battery according to the present invention connected in series. In addition, the structure of the battery stack can adopt any suitable structure and include any required devices, such as electrolyte tanks, circulation pumps and pipelines, etc., wherein single cells can be connected through pipelines, and electrolyte is stored in a storage tank, etc.

**Beneficial effect**

1. The present invention uses an ion exchange liquid membrane to replace the traditional expensive ion exchange solid membrane, which will greatly reduce the cost of the flow battery.
2. Traditional commercial ion exchange membranes limit proton conduction in terms of ion conduction, while ion exchange liquid membranes are not limited to protons in conducting ions, so they have wide applicability and can combine any two of organic, aqueous (including acidic, alkaline, and neutral), and aqueous-organic composite electrolytes, which greatly broadens the selection range of the electrode couple for batteries.
3. The ion exchange liquid membrane has adjustable thickness, which can effectively prevent crossover of active materials in the catholyte and the anolyte and improve the cycle performance of the battery.
4. The ion exchange liquid membrane has no fixed shape, thus is advantageous for multi-cell assembly, and facilitates the large-scale application.

**Description of the drawings**

Fig. 1 is a schematic diagram showing the design of single-cell structure of an ion exchange liquid membrane flow battery according to one embodiment of the present invention.

Fig. 2 is a charge-discharge curve diagram of the ion exchange liquid membrane flow battery based on ionic liquid in Example 1 of the present invention.

Fig. 3 is a cycle curve diagram of the ion exchange liquid membrane flow battery based on ionic liquid in Example

1 of the present invention.

Fig. 4 is a histogram of capacity utilization of the ion exchange liquid membrane flow battery based on ionic liquid in Example 2 of the present invention.

Fig. 5 is a cycle performance diagram of the ion exchange liquid membrane flow battery based on ionic liquid in Example 3 of the present invention.

Fig. 6 is a charge-discharge curve diagram of the ion exchange liquid membrane flow battery based on ionic liquid in Example 4 of the present invention.

Fig. 7 is a cycle performance diagram of the ion exchange liquid membrane flow battery based on ionic liquid in Example 4 of the present invention.

Fig. 8 is a charge-discharge curve diagram of the ion exchange liquid membrane flow battery based on ionic liquid in Example 5 of the present invention.

Fig. 9 is a cycle performance diagram of the ion exchange liquid membrane flow battery based on ionic liquid in Example 5 of the present invention.

Fig. 10 is a charge-discharge curve diagram of the ion exchange liquid membrane flow battery based on organic solvent in Example 6 of the present invention.

Fig. 11 is a cycle performance diagram of the ion exchange liquid membrane flow battery based on organic solvent in Example 6 of the present invention.

Fig. 12 is a cycle performance diagram of the ion exchange liquid membrane flow battery based on organic solvent in Example 7 of the present invention.

Fig. 13 is a charge-discharge curve diagram of the ion exchange liquid membrane flow battery based on water in Example 8 of the present invention.

Fig. 14 is a cycle performance diagram of the ion exchange liquid membrane flow battery based on water in Example 8 of the present invention.

## Detailed Embodiments

The terms used in this specification are only used to describe exemplary embodiments and are not intended to limit the present invention. Singular expressions may include plural expressions unless the context dictates a different expression. It should be understood that the terms "comprise", "contain" or "have" in this specification are only used to specify the presence of resulting features, numbers, steps, components or combinations thereof, and do not exclude the pre-existence or addition of one or multiple different features, numbers, steps, components, or combinations thereof.

The invention may be variously modified and have different forms, and specific examples of the invention are explained in detail below. However, there is no intention to limit the present invention to the specific examples, and it should be understood that the present invention includes all modifications, equivalents, or substitutions included within the spirit and technical scope of the present invention.

Examples are provided below for a better understanding of the present invention. However, the following examples are provided only for easier understanding of the present invention, and the scope of the present invention is not limited thereto.

## Examples

A flow battery was assembled using the structure shown in Fig. 1, in which a square tank with an internal size of 30*10*20 mm was used, the thickness of the intermediate bulkhead 1 was 1 mm, and the height of the bulkhead from the bottom is 5 mm. The entire container used acrylic plates for insulation. Current collectors were placed in the cathode and anode reaction chambers 2 and 3. The electrolytes were transported to the cathode and anode reaction chambers by the peristaltic pumps through pipelines 7 and 8.

The charge and discharge test was performed on a charge and discharge tester (Neware).

The charge and discharge capacity was obtained through constant current charge and discharge tests. The test was carried out in a constant temperature oven at a temperature of 25°C.

Coulombic efficiency was calculated as follows:

$$\text{Coulombic efficiency (CE)} = (\text{discharge capacity } (Q_d)/\text{charging capacity } (Q_c)) * 100\%$$

Energy efficiency was calculated as follows:

$$\text{Energy efficiency (EE)} = (\text{discharge energy } (E_d)/\text{charging energy } (E_c)) * 100\%$$

Capacity utilization was calculated as follows:

$$\text{Capacity utilization} = (\text{actual discharge capacity}/\text{theoretical discharge capacity}) * 100\%$$

The capacity decay rate was calculated as follows:

Capacity decay rate = (1- remaining discharge capacity after cycle/initial discharge capacity) * 100%

**Example 1**

An ionic liquid-based ion exchange liquid membrane redox flow battery was assembled by using a 5*5*10 mm graphite felt (purchased from Beihai Carbon) as the cathode, a 5*5*1 mm zinc plate as the anode, 2 mL of the ionic liquid $BMImPF_6$ added into the container as the ion exchange liquid membrane, a neutral aqueous solution as the anolyte comprising 1 mol/L $ZnCl_2$ as the anode active material, 1 mol/L $NH_4Cl$ as the pH adjustment functional additive and 0.1 mol/L BMImCl as the supporting solute, and a neutral aqueous solution as the catholyte comprising 0.001 mol/L $K_4Fe(CN)_6$ as the cathode active material and 0.1 mol/L BMImCl as the supporting solute.

The obtained battery was subjected to a constant current charge and discharge test at 0.1 mA. The charge and discharge cut-off voltages were 0.8 to 1.6 V, and the charge and discharge curve is shown in Fig. 2, demonstrating the operating voltage range and the good kinetic performance of the battery. Fig. 3 is the cycle performance of the obtained battery. After 100 cycles, the capacity retention is greater than 95%.

**Example 2**

An ion exchange liquid membrane redox flow battery was assembled in the same manner as in Example 1, except that the catholyte was adjusted to an alkaline aqueous solution containing 0.1 mol/L $K_4Fe(CN)_6$ as the cathode active material, 1 mol/L KOH as the pH adjustment functional additive and 0.1 mol/L BMImCl as the supporting solute.

The obtained battery was charged and discharged at a current of 0.1C, 0.2C (0.268 mA, 0.536 mA) respectively, and the charge and discharge cut-off voltages were 0.75 to 1.55 V Fig. 4 shows the charge and discharge capacity utilizationof the obtained battery at the corresponding currents, both of which were around 70%.

**Example 3**

An ion exchange liquid membrane redox flow battery was assembled in the same manner as in Example 1, except that the catholyte was adjusted to an alkaline aqueous solution containing 0.03 mol/L $K_4Fe(CN)_6$ as the cathode active material, 1 mol/L KOH as the pH adjustment functional additive, and 0.1 mol/L BMImCl as the supporting solute.

The obtained battery was subjected to a constant current charge and discharge test at 0.1 mA. The results are shown in Fig. 5. The 60-day capacity decay of the obtained battery was less than 7%.

**Example 4**

An ion exchange liquid membrane redox flow battery was assembled in the same manner as in Example 1, except that a graphite felt (purchased from Beihai Carbon) was used as the current collector for anode, an aqueous solution comprising 0.01 mol/L DHBQ as the anode active material and 0.1 mol/L BMImCl as the supporting solute was used as the anolyte, and an aqueous solution comprising 0.02 mol/L $K_4Fe(CN)_6$ and 0.01 mol/L $K_3Fe(CN)_6$ as the cathode active material and 0.1 mol/L BMImCl as the supporting solute was used as the catholyte.

The obtained battery was subjected to a constant current charge and discharge test at 0.5 mA. The charge and discharge cut-off voltages were 0.1 to 1.3 V The charge and discharge curve is shown in Fig. 6.

Fig. 7 shows the cycle performance of the obtained battery. The coulombic efficiency was about 88%. After 10 cycles, the capacity retention was 85%.

**Example 5**

An ion exchange liquid membrane redox flow battery was assembled in the same manner as in Example 1, except that a graphite felt was used as the current collector for anode, an aqueous solution comprising 0.01 mol/L 2,6-DHAQ as the anode active material and 0.1 mol/L BMImCl as the supporting solute was used as the anolyte, and an

aqueous solution comprising 0.001 mol/L $K_4Fe(CN)_6$ as the cathode active material and 0.1 mol/L BMImCl as the supporting solute was used as the catholyte.

The obtained battery was subjected to a constant current charge and discharge test at 0.2 mA. The charge and discharge cut-off voltages were 0.6 to 1.65 V The charge and discharge curve is shown in Fig. 8.

Fig. 9 shows the cycle performance of the obtained battery. After 50 cycles, the average capacity decay rate per cycle was less than 0.5%.

**Example 6**

An organic solvent-based ion exchange liquid membrane redox flow battery was assembled by using a 5*5*10 mm graphite felt (purchased from Beihai Carbon) as the cathode, and a 5*5*1 mm zinc plate as the anode.

The catholyte was prepared as follows: a mixture of water and triethyl phosphate (TEP) with a volume ratio of 2:1 was added in another container, and 0.167 g/mL $(NH_4)_2SO_4$ as the salting-out functional additive was added thereto to utilize the salting-out effect to separate the water and TEP. To the aqueous layer, 0.01 mol/L $K_4Fe(CN)_6$ and 0.005 mol/L $K_3Fe(CN)_6$ as the cathode active material, and 0.167 g/mL LiTFSI as the supporting solute were added. Under this condition, the TEP layer was heavier than the aqueous layer. After the stratifying was stablized, the aqueous layer was taken as the catholyte.

The anolyte was prepared as follows: a mixture of water and triethyl phosphate (TEP) with a volume ratio of 2:1 was added in another container, and 0.167 g/mL $(NH_4)_2SO_4$ as the salting-out functional additive was added thereto to utilize the salting-out effect to separate the water and TEP. To the aqueous layer, 0.03 mol/L $ZnSO_4$ as the anode active material and 0.167 g/mL LiTFSI as the supporting solute were added. Under this condition, the TEP layer was heavier than the water layer. After the stratifying was stabilized, the water layer was taken as the anolyte.

Ion exchange liquid membrane: The TEP layer obtained during the preparation of the catholyte and anolyte was taken as the ion exchange liquid membrane.

When assembling the battery, 2 mL of the ion exchange liquid membrane was firstly added to the container, and then the catholyte and anolyte were added to the cathode and anode chambers respectively. The obtained battery was subjected to a constant current charge and discharge test at 0.2 mA. The charge and discharge cut-off voltages were 1.0 to 1.7V. The charge and discharge curve is shown in Fig. 10.

Fig. 11 shows the cycle performance of the obtained battery. The average capacity decay rate per cycle within 50 cycles was about 0.1%, the Coulombic efficiency was greater than 97%, and the energy efficiency was close to 90%.

**Example 7**

An ion exchange liquid membrane redox flow battery was assembled in the same manner as in Example 6, except that the cathode active material was changed to 0.01 mol/L $K_4Fe(CN)_6$ and 0.005 mol/L $K_3Fe(CN)_6$; and the anode active material was changed to 0.1 mol/L $ZnSO_4$.

The obtained battery was subjected to a constant current charge and discharge test at 0.5 mA. The charge and discharge cut-off voltages were 1.0 to 1.8V

Fig. 12 shows the cycle curve of the obtained battery. There was almost no decay in capacity within 50 cycles, the Coulombic efficiency was around 100%, and the energy efficiency was greater than 70%.

**Example 8**

A water-based ion exchange liquid membrane redox flow battery was assembled by using a 5*5*10 mm graphite felt (purchased from Beihai Carbon) as the cathode, and a 5*5*1 mm zinc plate as the anode.

The catholyte was prepared as follows: a mixture of water and triethyl phosphate (TEP) with a volume ratio of 1:1 was added in another container, and 0.28 g/mL $(NH_4)_2SO_4$ as the salting-out functional additive was added thereto to utilize the salting-out effect to separate the water and TEP. To the TEP layer, 0.04 mol/L TEMPO as the cathode active material and 0.25 g/mL LiTFSI as the supporting solute were added. Under this condition, the aqueous layer was heavier than the TEP layer. After the stratifying was stablized, the TEP layer was taken as the catholyte.

The anolyte was prepared as follows: a mixture of water and triethyl phosphate (TEP) with a volume ratio of 1:1 was added in another container, and 0.28 g/mL $(NH_4)_2SO_4$ as the salting-out functional additive was added thereto to utilize the salting-out effect to separate the water and TEP. To the TEP layer, 0.01 mol/L $Zn(TFSI)_2$ as the anode active material and 0.25 g/mL LiTFSI as the supporting solute were added. Under this condition, the aqueous layer was heavier than the TEP layer. After the stratifying was stablized, the TEP layer was taken as the anolyte.

Ion exchange liquid membrane: The water layer obtained during the preparation of the catholyte and anolyte was taken as the ion exchange liquid membrane.

When assembling the battery, 2 mL of the ion exchange liquid membrane was firstly added to the container,

and then the catholyte and anolyte were added to the cathode and anode chambers respectively. The obtained battery was subjected to a constant current charge and discharge test at 0.2 mA. The charge and discharge cut-off voltages were 1.3 to 1.8 V. The charge and discharge curve is shown in Fig. 13.

Fig. 14 shows the cycle performance of the obtained battery. There was almost no decay in capacity within 15 cycles, the Coulombic efficiency was around 85%, and the energy efficiency was 68%.

It can be seen from the results that the ion exchange liquid membrane technology for flow batteries provided by the present invention not only has a reasonable electrochemical theoretical basis, but its applicability can be proven through various examples. The above examples demonstrate that, when the present invention uses an ionic liquid, an organic solvent, or water as the ion exchange liquid membrane, other necessary conditions for the operation of the flow battery, including the composition of the catholyte and anolyte, the pH value, the types of cathode and anode, the battery structure and other factors, can be freely replaced according to requirements, showing a strong versatility. In addition, the electrochemical data provided in the examples show that the ion exchange liquid membrane provided by the present invention exhibits reasonably good electrochemical performance, which is specifically exhibited by reasonably high Coulombic efficiency, energy efficiency and cycling stability during the charge and discharge cycle, when applied to a flow battery.

## Claims

1. A flow battery including an ion exchange liquid membrane, wherein the ion exchange liquid membrane separates catholyte and anolyte by independently stratifying with the catholyte and the anolyte, and it dissolves a supporting solute but does not dissolve cathode and anode active materials.

2. The flow battery according to claim 1, wherein the ratio of the solubility of the cathode active material in the catholyte to the solubility in the ion exchange liquid membrane, and the ratio of the solubility of the anode active material in the anolyte to the solubility in the ion exchange liquid membrane are independently greater than 10.

3. The flow battery according to claim 1, wherein the ion exchange liquid membrane is an ionic liquid-based, water-based or organic solvent-based liquid.

4. The flow battery according to claim 3, comprising:

   an ion exchange liquid membrane, which is an ionic liquid-based, water-based or organic solvent-based liquid;
   a catholyte, comprising a solvent-I, a cathode active material, a supporting solute and optionally, a functional additive;
   an anolyte, comprising a solvent-II, an anode active material, the supporting solute and optionally, a functional additive;
   wherein the ion exchange liquid membrane separates the catholyte and the anolyte by independently stratifying with the catholyte and the anolyte, and it dissolves the supporting solute but does not dissolve the cathode and anode active materials.

5. The flow battery according to claim 4, comprising:

   a catholyte, comprising a solvent-I, a cathode active material, a supporting solute, and optionally, a functional additive;
   an anolyte, comprising a solvent-II, an anode active material, the supporting solute, and optionally, a functional additive;
   the ion exchange liquid membrane, which is an ionic liquid-based liquid, separates the catholyte and anolyte by independently stratifying with the catholyte and the anolyte, dissolves the supporting solute, but does not dissolve the cathode and anode active materials.

6. The flow battery according to any one of claims 3 to 5, wherein the ionic liquid is selected from a hexafluorophosphate-containing ionic liquid, a tetrafluoroborate-containing ionic liquid, a perchlorate-containing ionic liquid, a bis(trifluoromethylsulfonyl)imide-containing ionic liquid, a bisfluorosulfonimide-containing ionic liquid, a trifluoroacetate-containing ionic liquid, a dinitrile amine-containing ionic liquid, a trifluoromethanesulfonate-containing ionic liquid, a thiocyanate-containing ionic liquid, and a mixture thereof.

7. The flow battery according to any one of claims 3 to 5, wherein the ionic liquid is selected from 1-butyl-3-methylimidazolium hexafluorophosphate, 1-ethyl-3-methylimidazolium tetrafluoroborate, 1 -propyl-3 -methylimidazolium tetrafluoroborate, 1-butyl-3-methylimidazolium tetrafluoroborate, 1-ethyl-3-methylimidazolium perchlorate, 1-propyl-3-methylimidazolium perchlorate, 1-butyl-3-methylimidazolium perchlorate, 1-ethyl-3-methylimidazolium bis(trifluoromethylsulfonyl)imide, 1-propyl-3-methylimidazolium bis(trifluoromethylsulfonyl)imide, 1-butyl-3-methylimidazolium bis(trifluoromethylsulfonyl)imide.

**8.** The flow battery according to claim 4 or 5, wherein the solvent-I and solvent-II are each independently selected from water, a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound and a mixture thereof.

**9.** The flow battery according to claim 4 or 5, wherein the solvent-I and solvent-II are each independently selected from water, trimethyl phosphate, triethyl phosphate, triphenyl phosphate, tris(2,2,2-trifluoroethyl) phosphate, N,N-dimethylformamide, N,N-dimethylacetamide, N-methylpropionamide, trifluoroacetamide, N-methyltrifluoroacetamide, N,N-dimethyltrifluoroacetamide, hexamethyldisiloxane, hexa(trifluoropropyl)disiloxane, trifluoropropylmethylcyclotrisiloxane, aminopropyl pentamethyl disiloxane, hydroxymethyl pentamethyl disiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, and a mixture thereof.

**10.** The flow battery according to claim 4 or 5, wherein

the cathode active material is selected from:

(1) potassium ferrocyanide, having a chemical formula of $K_4Fe(CN)_6$, an oxidized state of ferricyanide ion ($[Fe(CN)_6]^{3-}$), and a reduced state of ferrocyanide ion ($[Fe(CN)_6]^{4-}$);
(2) a 2,2,6,6-tetramethylpiperidine oxide compound, having an oxidized state shown in the following formula 2, and a reduced state shown in the following formula 1,

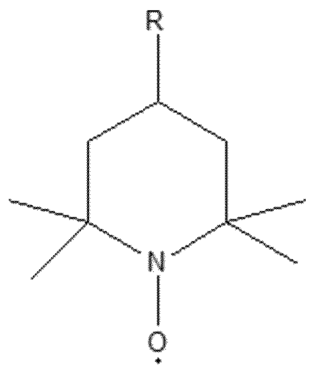

1

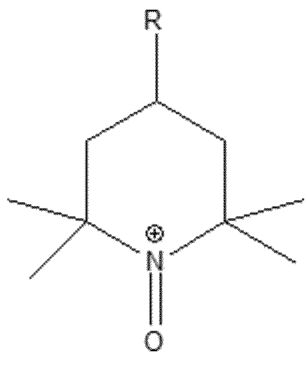

2

wherein R is selected from hydrogen, hydroxyl, carboxyl, halogen, C1 to C20 alkyl, C1 to C20 alkoxy, amino,

C1 to C20 alkylamino, carboxyl C1 to C20 alkylamino, C1 to C20 amide group, C6 to C20 aryl, and sulfo;
(3) a ferrocene compound, having an oxidized state shown in the following formula 4 and a reduced state shown in the following formula 3,

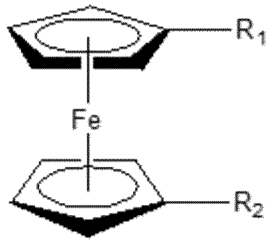

3

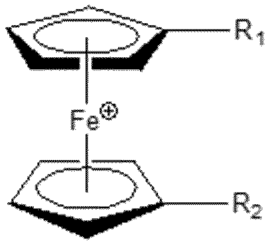

4

wherein $R_1$ and $R_2$ are each independently selected from hydrogen, hydroxyl, carboxyl, halogen, C1 to C20 alkyl, C1 to C20 alkoxy, amino, C 1 to C20 alkylamino, carboxyl C 1 to C20 alkylamino, C1 to C20 amide group, C6 to C20 aryl, and sulfo;
(4) vanadium(IV) oxide sulfate, having a chemical formula of $VOSO_4$, an oxidized state of pentavalent vanadium ion, and a reduced state of tetravalent vanadium ion;
(5) a metal iodide ($MI_n$) or hydrogen iodide (HI), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $I_2$ and the reduced state is $MI_n$ or HI;
(6) 2,5-dimercaptothiadiazole, having a molecular formula of $C_2H_2N_2S_3$, an oxidized state of $(C_2HN_2S_3)_2$, and a reduced state of $C_2H_2N_2S_3$;
(7) a catechol organic compound, having an oxidized state shown in the following formula 6, and a reduced state shown in the following formula 5,

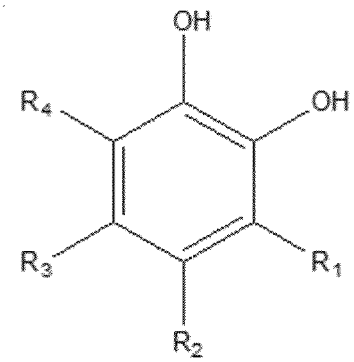

5

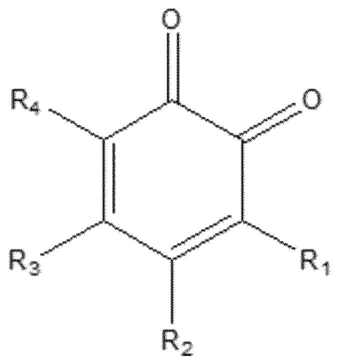

6

wherein $R_1$, $R_2$, $R_3$, and $R_4$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo;

(8) a divalent iron salt, having a chemical formula of $FeX_n$, wherein X is selected from $Cl^-$, Br, $SO_4^{2-}$, $NO_3^-$, and $ClO_4^-$, n is 1 or 2, the oxidized state is trivalent iron ion ($Fe^{3+}$), and the reduced state is divalent iron ion ($Fe^{2+}$);

(9) a metal bromide ($MBr_n$) or hydrogen bromide (HBr), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $Br_2$, and the reduced state is $MBr_n$ or HBr;

the anode active material is selected from:

(1) a divalent zinc salt, having a chemical formula of $ZnX_n$, wherein X is selected from $Cl^-$, Br, $SO_4^{2-}$, $NO_3^-$, $CH_3COO^-$, $TFSI^-$, and OTf, n is 1 to 2, the oxidized state is zinc ion ($Zn^{2+}$), and the reduced state is metallic zinc;

(2) methyl viologen (MV), having a molecular formula of $C_{12}H_{14}Cl_2N_2$, an oxidized state of $MV^{2+}$, and a reduced state of $MV^+$;

(3) an acetylacetone metal salt, having a chemical formula of $M(acac)_n$, an oxidized state of metal ion ($M^{n+}$), and a reduced state of metal element M or a lower-valent acetylacetone metal salt $M^{m+}$, wherein M is selected from Li, Fe, Al, Ni, Be, Ga, In, V, Cr, Co, Mn, Cu, Pt, Ru, Ti, Cd, and Mo, n is an integer from 1 to 4, m is an integer from 1 to 3, and m<n;

(4) a p-benzoquinone compounds, having an oxidized state shown in the following formula 7 and a reduced state shown in the following formula 8:

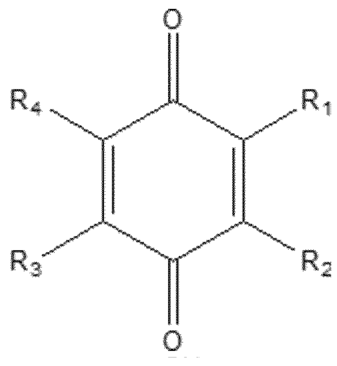

7

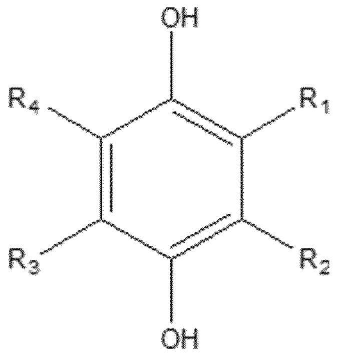

8

wherein $R_1$, $R_2$, $R_3$, and $R_4$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo;

(5) an anthraquinone compound, having an oxidized state shown in the following formula 9, and a reduced state shown in the following formula 10:

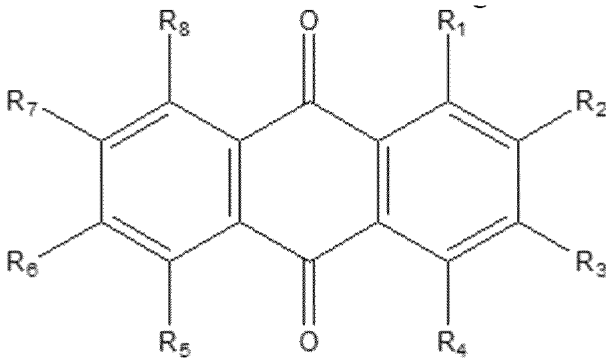

9

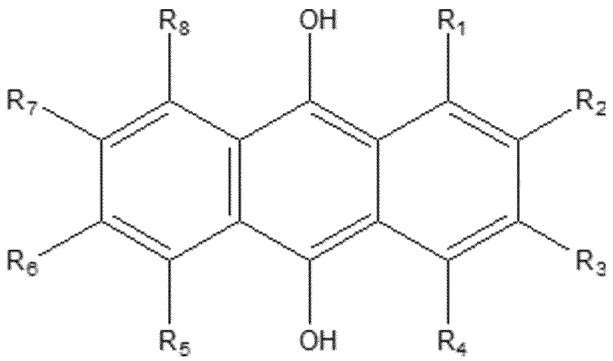

10

wherein $R_1$, $R_2$, $R_3$, $R_4$, $R_5$, $R_6$, $R_7$, and Rs are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo;

(6) a phenazine compound, having an oxidized state shown in the following formula 11, and a reduced state shown in the following formula 12:

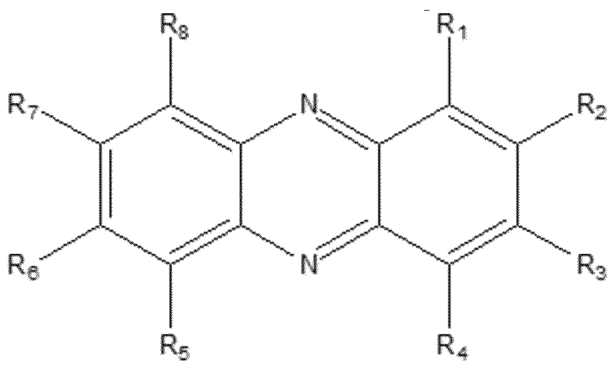

11

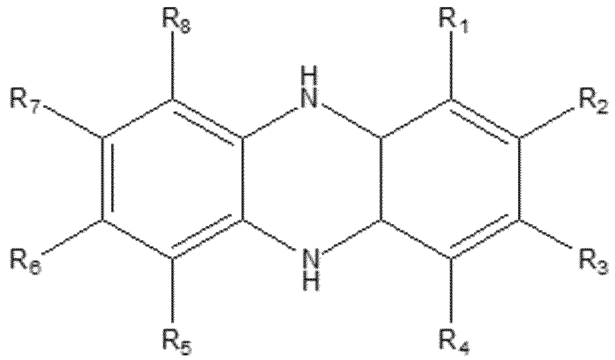

12

wherein $R_1$, $R_2$, $R_3$, $R_4$, $R_5$, $R_6$, $R_7$, and Rs are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo;

(7) vanadium sulfate (III), having a chemical formula of $V_2(SO_4)_3$, an oxidized state of trivalent vanadium ion ($V^{3+}$), and a reduced state of divalent vanadium ion ($V^{2+}$);

(8) camphorquinone, having a molecular formula of $C_{10}H_{14}O_2$, an oxidized state of $C_{10}H_{14}O_2$, and a reduced state of $C_{10}H_{14}O_2^{-}$;

(9) N-methylphthalimide, having a molecular formula of $C_9H_7NO_2$, an oxidized state of $C_9H_7NO_2$, and a reduced state of $C_9H_7NO_2^{-}$;

(10) a fluorenone compound, having an oxidized state shown in the following formula 13, and a reduced state shown inthe following formula 14:

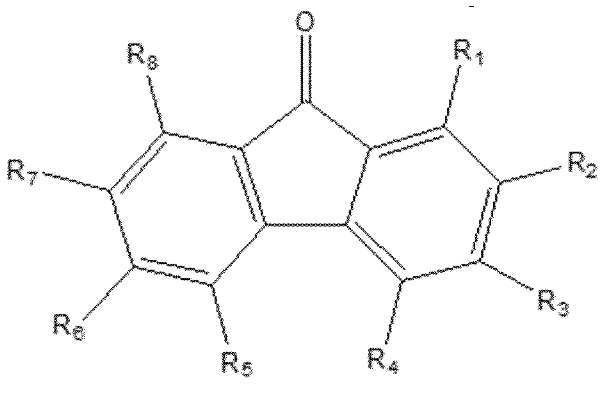

13

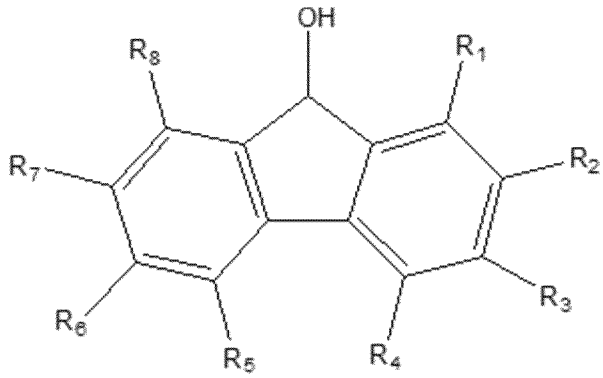
14

wherein $R_1$, $R_2$, $R_3$, $R_4$, $R_5$, $R_6$, $R_7$, and Rs are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo;

(11) a trivalent chromium salt, having a chemical formula of $CrX_n$, where X is selected from $Cl^-$, Br, $SO_4^{2-}$, $NO_3^-$, and $ClO_4^-$, n is 1.5 or 3, and the oxidized state is trivalent chromium ion ($Cr^{3+}$), and the reduced state is divalent chromium ion ($Cr^{2+}$).

**11.** The flow battery according to claim 4, comprising:

a catholyte, comprising water, a cathode active material, a supporting solute and optionally, a functional additive;
an anolyte, comprising water, an anode active material, the supporting solutes and optionally, a functional additive;
the ion exchange liquid membrane, which is an organic solvent-based liquid, separates the catholyte and the anolyte by independently stratifying with the catholyte and the anolyte, and dissolves the supporting solute but does not dissolve the cathode and anode active materials, thereby providing a pathway for the transport of the supporting solute ions between the catholyte and the anolyte.

**12.** The flow battery according to claim 11, wherein

the cathode active material is selected from:

(1) potassium ferrocyanide, having a chemical formula of $K_4Fe(CN)_6$, an oxidized state of ferricyanide ion ($[Fe(CN)_6]^{3-}$), and a reduced state of ferrocyanide ion ($[Fe(CN)_6]^{4-}$);

(2) a water-soluble 2,2,6,6-tetramethylpiperidine oxide compound, having an oxidized state shown in the following formula 2, and a reduced state shown in the following formula 1,

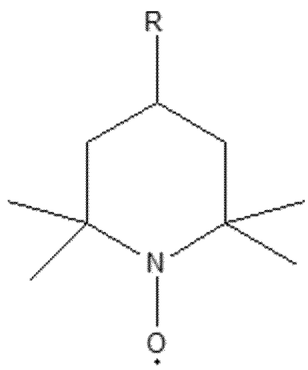
1

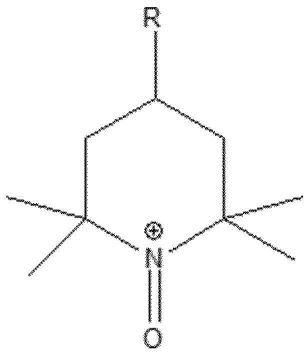

2

wherein R is selected from hydroxyl, carboxyl, amino, carboxyl C1 to C20 alkylamino, C1 to C20 amide group, and sulfo;

(3) a water-soluble ferrocene compound, having an oxidized state shown in the following formula 4, and a reduced state shown in the following formula 3:

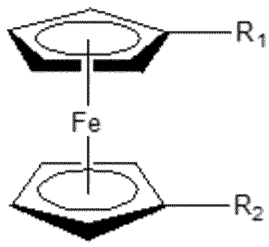

3

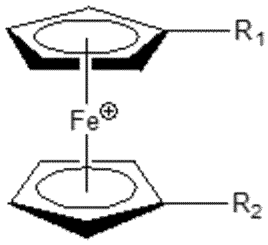

4

wherein Ri and $R_2$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$ and $R_2$ is selected from hydroxyl, carboxyl, amino, carboxyl C1 to C20 alkylamino, C1 to C20 amide group, and sulfo;

(4) vanadium(IV) oxide sulfate, having a chemical formula is $VOSO_4$, an oxidized state of pentavalent vanadium ion, and a reduced state of tetravalent vanadium ion;

(5) a metal iodide ($MI_n$) or hydrogen iodide (HI), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $I_2$ and the reduced state is $MI_n$ or HI;

(6) a water-soluble catechol organic compound, having an oxidized state shown in the following formula 6, and an reduced state shown in the following formula 5:

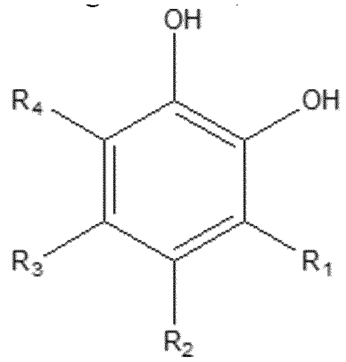

5

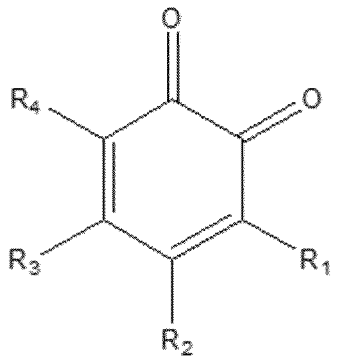

6

wherein $R_1$, $R_2$, $R_3$, and $R_4$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$, $R_2$, $R_3$, and $R_4$ is selected from hydroxyl, carboxyl, amino, carboxyl C1 to C20 alkylamino, C1 to C20 amide group, and sulfo;

(7) a divalent iron salt, having a chemical formula of $FeX_n$, wherein X is selected from $Cl^-$, Br, $SO_4^{2-}$, $NO_3^-$, $ClO_4^-$, n is 1 or 2, and the oxidized state is trivalent iron ion ($Fe^{3+}$), the reduced state is divalent iron ion ($Fe^{2+}$);

(8) a metal bromide ($MBr_n$) or hydrogen bromide (HBr), wherein M is selected from Li, Na, K, Rb, Cs, Mg, Ca, Fe, Al, Ni, Be, Ga, In, V, Cr, Co, Mn, Cu, Pt, Ru, Ti, Cd, and Mo, n is an integer from 1 to 4, the oxidized state is $Br_2$, and the reduced state is $MBr_n$ or HBr;

the anode active material is selected from:

(1) a water-soluble divalent zinc salt, having a chemical formula of $ZnX_n$, wherein X is selected from $Cl^-$, Br, $SO_4^{2-}$, $NO_3^-$, and $CH_3COO^-$, n is 1 to 2, the oxidized state is zinc ion ($Zn^{2+}$), and the reduced state is metallic zinc;

(2) methyl viologen (MV), having a molecular formula of $C_{12}H_{14}Cl_2N_2$, an oxidized state of $MV^{2+}$, and a reduced state of $MV^+$;

(3) a water-soluble p-benzoquinone compound, having an oxidized state shown in the following formula 7, and a reduced state shown in the following formula 8:

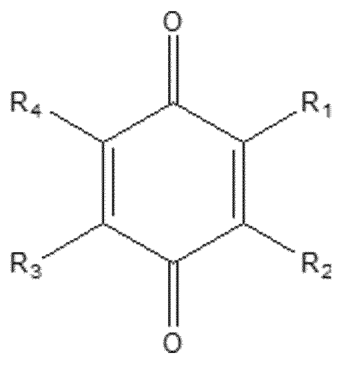

7

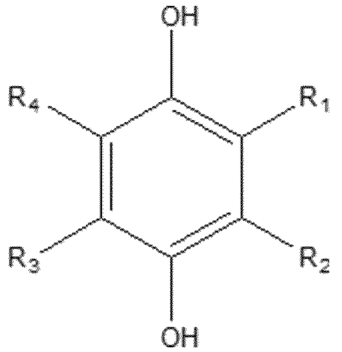

8

wherein $R_1$, $R_2$, $R_3$, and $R_4$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$, $R_2$, $R_3$, and $R_4$ is selected from hydroxyl, carboxyl, amino, carboxyl C1 to C20 alkylamino, C1 to C20 amide group, and sulfo;

(4) a water-soluble anthraquinone compound, having an oxidized state shown in the following formula 9, and a reduced state shown in the following formula 10:

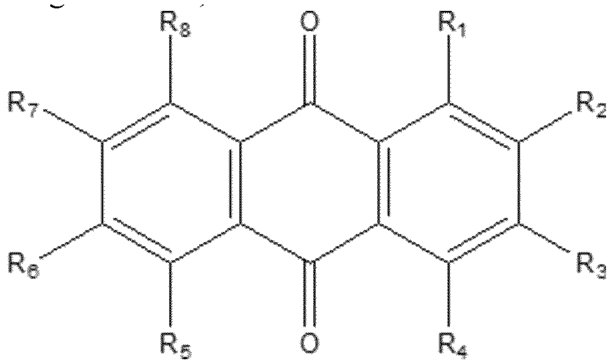

9

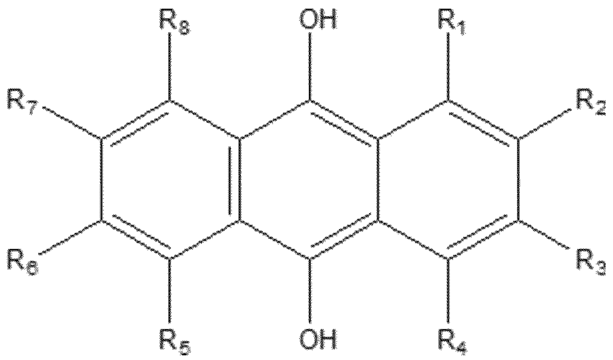

10

wherein $R_1$, $R_2$, $R_3$, $R_4$, $R_5$, $R_6$, $R_7$, and Rs are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$, $R_2$, $R_3$, $R_4$, $R_5$, $R_6$, $R_7$, and Rs is selected from hydroxyl, carboxyl, amino, carboxyl C1 to C20 alkylamino, C1 to C20 amide group, and sulfo;

(5) a water-soluble phenazine compound, having an oxidized state shown in the following formula 11, and a reduced state shown in the following formula 12:

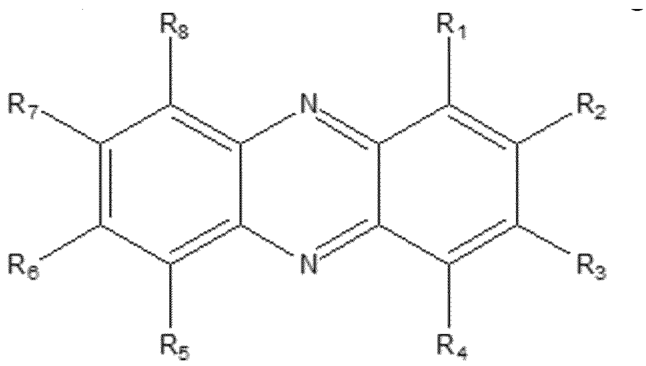

11

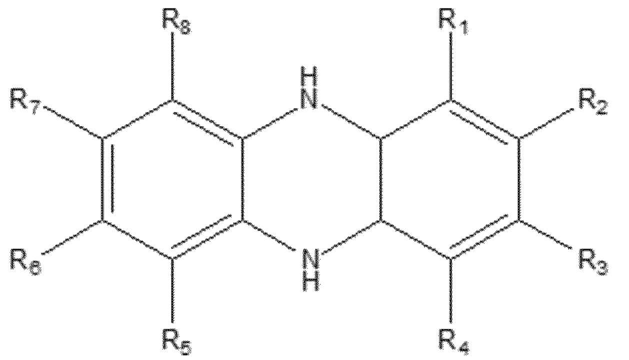

12

wherein $R_1$, $R_2$, $R_3$, $R_4$, $R_5$, $R_6$, $R_7$, and $R_8$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$, $R_2$, $R_3$, $R_4$, $R_5$, $R_6$, $R_7$, and $R_8$ is selected from hydroxyl, carboxyl, amino, carboxyl C1 to C20 alkylamino, C1 to C20 amide group, and sulfo;

(6) vanadium sulfate (III), having a chemical formula of $V_2(SO_4)_3$, an oxidized state of trivalent vanadium ion ($V^{3+}$), and a reduced state of divalent vanadium ion ($V^{2+}$);

(7) a water-soluble fluorenone compound, having an oxidized state shown in the following formula 13, and a reduced state shown in the following formula 14:

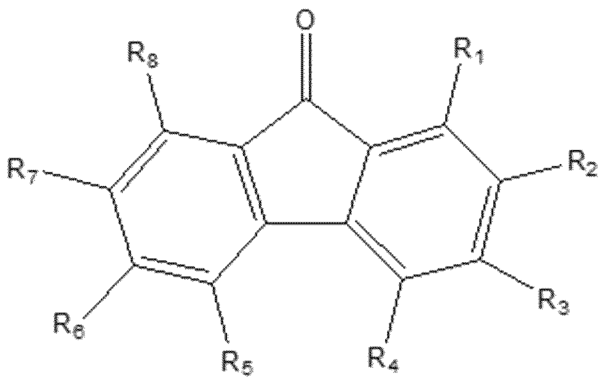

13

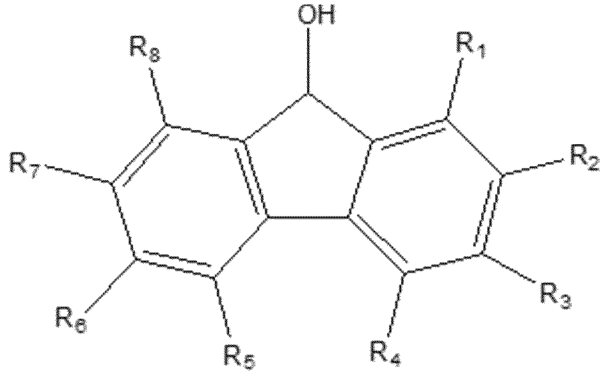

14

wherein $R_1$, $R_2$, $R_3$, $R_4$, $R_5$, $R_6$, $R_7$, and $R_8$ are each independently selected from hydrogen, hydroxyl, carboxyl, halogen, C1 to C20 alkyl, C1 to C20 alkoxy, amino, C1 to C20 alkylamino, carboxyl C1 to C20 alkylamino, C1 to C20 amide group, C6 to C20 aryl, and sulfo; and at least one of $R_1$, $R_2$, $R_3$, $R_4$, $R_5$, $R_6$, $R_7$, and $R_8$ is selected from hydroxyl, carboxyl, amino, carboxyl C1 to C20 alkylamino, C1 to C20 amide group, and sulfo;

(8) a trivalent chromium salt, having a chemical formula of $CrX_n$, wherein X is selected from $Cl^-$, Br, $SO_4^{2-}$, $NO_3^-$, and $ClO_4^-$, n is 1.5 or 3, and the oxidized state is trivalent chromium ion ($Cr^{3+}$), the reduced state is divalent chromium ion ($Cr^{2+}$).

**13.** The flow battery according to claim 4, comprising:

a catholyte, comprising an organic solvent-I, a cathode active material, a supporting solute and optionally, a functional additive;

an anolyte, comprising an organic solvent-II, an anode active material, the supporting solute and optionally, a functional additive;

the ion exchange liquid membrane, which is a water-based liquid, separates the catholyte and anolyte by independently stratifying with the catholyte and the anolyte, and dissolves the supporting solute but does not dissolve the cathode and anode active materials.

**14.** The flow battery according to claim 13, wherein

the cathode active material is selected from:

(1) a water-insoluble 2,2,6,6-tetramethylpiperidine oxide compound, having an oxidized state shown in the following formula 2, and a reduced state shown in the following formula 1,

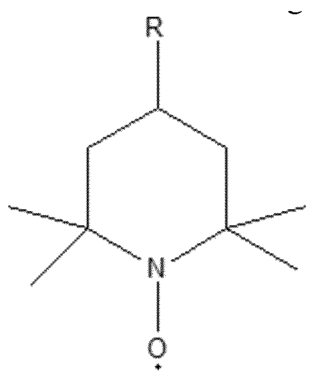

1

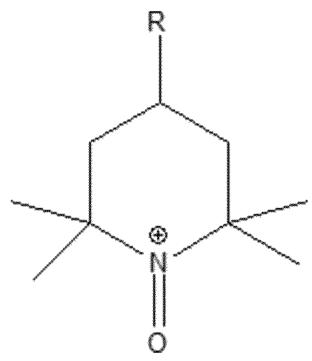

2

wherein R is selected from hydrogen, halogen, C1 to C20 alkyl, C1 to C20 alkoxy, C1 to C20 alkylamino, and C6 to C20 aryl;

(2) a water-insoluble ferrocene compound, having an oxidized state shown in the following formula 4, and a reduced state shown in the following formula 3:

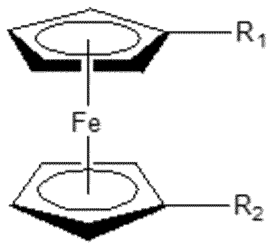

3

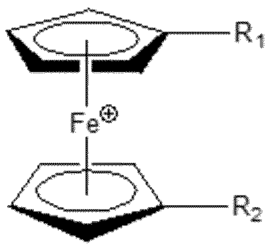

4

wherein $R_1$ and $R_2$ are each independently selected from hydrogen, halogen, C1 to C20 alkyl, C1 to C20 alkoxy, C1 to C20 alkylamino, and C6 to C20 aryl;

(3) 2,5-dimercaptothiadiazole, having a molecular formula of $C_2H_2N_2S_3$, an oxidized state of $(C_2HN_2S_3)_2$, and a reduced state of $C_2H_2N_2S_3$;

(4) a water-insoluble catechol-based organic compound, having an oxidized state shown in the following formula 6, and a reduced state shown in the following formula 5:

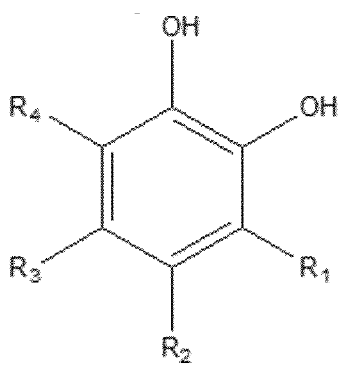

5

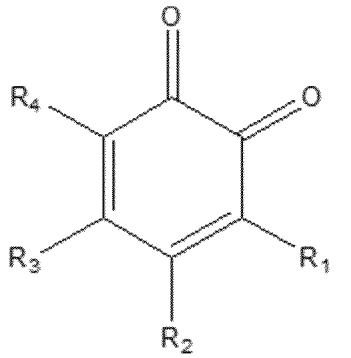

6

wherein $R_1$, $R_2$, $R_3$ and $R_4$ are each independently selected from hydrogen, halogen, C1 to C20 alkyl, C1 to C20 alkoxy, C1 to C20 alkylamino, and C6 to C20 aryl;

the anode active material is selected from:

(1) a water-insoluble divalent zinc salt, having a chemical formula of $ZnX_2$, wherein X is selected from $TFSI^-$ and $OTf^-$, the oxidized state is zinc ion ($Zn^{2+}$), and the reduced state is metallic zinc;
(2) an acetylacetone metal salt, having a chemical formula of $M(acac)_n$, an oxidized state of metal ion ($M^{n+}$), and a reduced state of metal element (M) or a lower-valent acetylacetone metal salt ($M^{m+}$), wherein M is selected from Li, Fe, Al, Ni, Be, Ga, In, V, Cr, Co, Mn, Cu, Pt, Ru, Ti, Cd, and Mo, n is an integer from 1 to 4, m is an integer from 1 to 3, and m<n;
(3) a water-insoluble p-benzoquinone compound, having an oxidized state shown in the following formula 7, and a reduced state shown in the following formula 8:

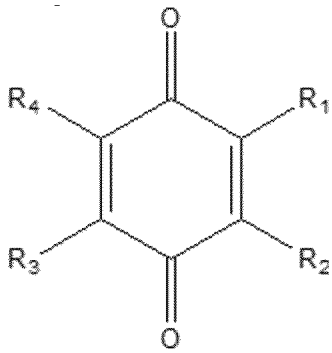

7

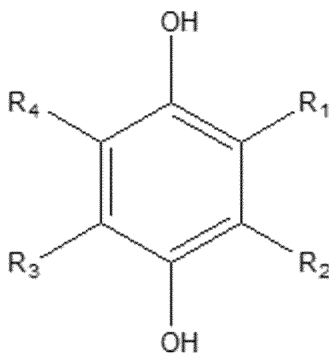

8

wherein $R_1$, $R_2$, $R_3$ and $R_4$ are each independently selected from hydrogen, halogen, C1 to C20 alkyl, C1 to C20 alkoxy, C1 to C20 alkylamino, and C6 to C20 aryl;
(4) a water-insoluble anthraquinone compound, having an oxidized state shown in the following formula 9, and a reduced state shown in the following formula 10:

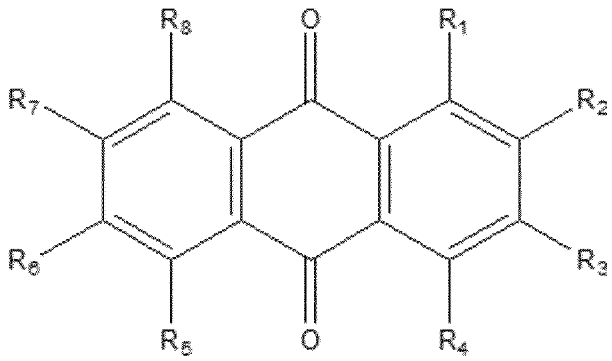

9

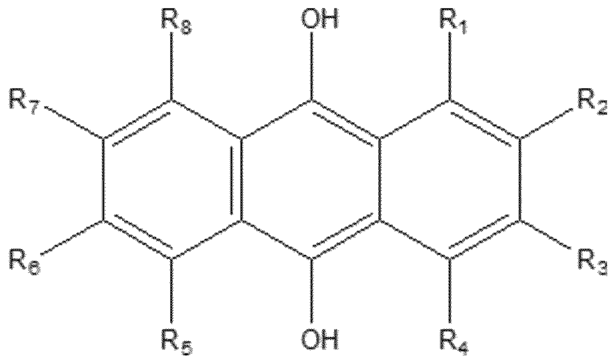

10

wherein $R_1$, $R_2$, $R_3$, $R_4$, $R_5$, $R_6$, $R_7$, and $R_8$ are each independently selected from hydrogen, halogen, C1 to C20 alkyl, C1 to C20 alkoxy, C1 to C20 alkylamino, and C6 to C20 aryl;
(5) a water-insoluble phenazine compound, having an oxidized state shown in the following formula 11, and a reduced state shown in the following formula 12:

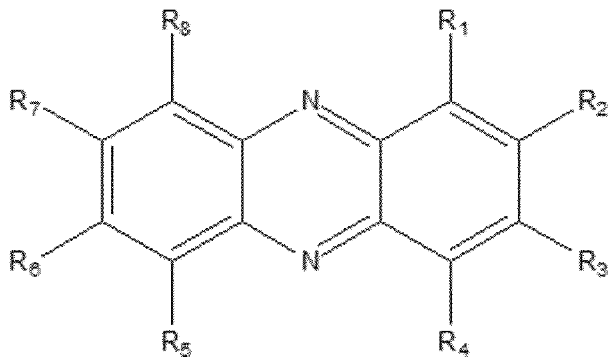

11

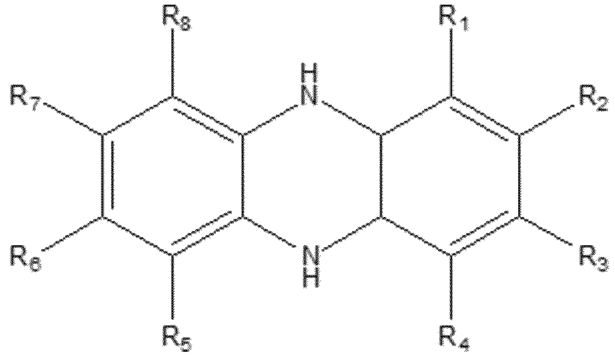

12

wherein $R_1$, $R_2$, $R_3$, $R_4$, $R_5$, $R_6$, $R_7$, and $R_8$ are each independently selected from hydrogen, halogen, C1

to C20 alkyl, C1 to C20 alkoxy, C1 to C20 alkylamino, and C6 to C20 aryl;

(6) camphorquinone, having a molecular formula of $C_{10}H_{14}O_2$, an oxidized state of $C_{10}H_{14}O_2$, and a reduced state of $C_{10}H_{14}O_2^{-}$;

(7) N-methylphthalimide, having a molecular formula is $C_9H_7NO_2$, an oxidized state of $C_9H_7NO_2$, and a reduced state of $C_9H_7NO_2^{-}$;

(8) a water-insoluble fluorenone compound, having an oxidized state shown in the following formula 13, and a reduced state shown in the following formula 14:

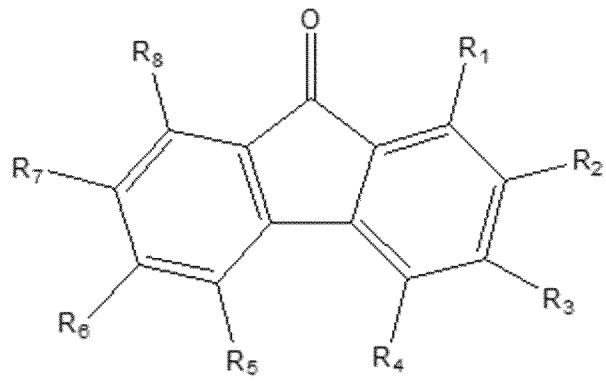

13

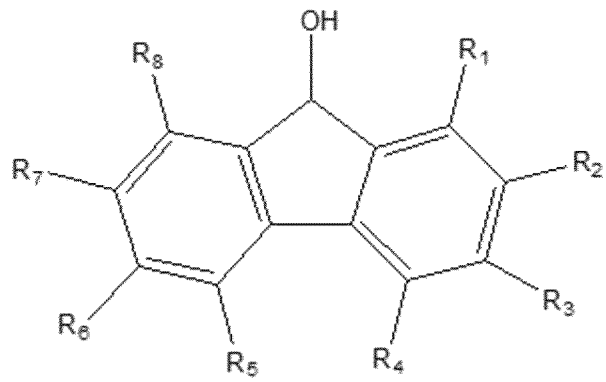

14

wherein $R_1$, $R_2$, $R_3$, $R_4$, $R_5$, $R_6$, $R_7$, and $R_8$ are each independently selected from hydrogen, halogen, C1 to C20 alkyl, C1 to C20 alkoxy, C1 to C20 alkylamino, and C6 to C20 aryl.

**15.** The flow battery according to claim 3, 4, 11 or 13, wherein the organic solvent is selected from a phosphate compound, an amide compound, a siloxane compound, a carbonate compound, a sulfate compound, a sulfite compound, a carboxylate compound, a ketone compound, an ether compound, a sulfone compound, an alcohol compound, a nitrile compound, a pyridine compound, a halogenated hydrocarbon compound, an aromatic compound, and a mixture thereof.

**16.** The flow battery according to claim 3, 4, 11 or 13, wherein the organic solvent is selected from trimethyl phosphate, triethyl phosphate, triphenyl phosphate, tris(2,2,2-trifluoroethyl) phosphate, N,N-dimethylformamide, N,N-dimethylacetamide, N-methylpropionamide, N-methyltrifluoroacetamide, N,N-dimethyltrifluoroacetamide, hexamethyldisiloxane, hexa(trifluoropropyl)disiloxane, trifluoropropylmethylcyclotrisiloxane, dimethyl carbonate, ethyl methyl carbonate, diethyl carbonate, ethylene carbonate, propylene carbonate, fluoroethylene carbonate, dimethyl sulfate, vinyl sulfite, ethyl acetate, 1,4-butyrolactone, ethylene glycol monomethyl ether, ethylene glycol dimethyl ether, tetraethylene glycol dimethyl ether, 4-methyl-2-pentanone, tetrahydrofuran, 2-methyltetrahydrofuran, 1,3-dioxolane, dimethyl sulfoxide, acetonitrile, pyridine, 1,2-dichloroethane, nitrobenzene, and a mixture thereof.

**17.** The flow battery according to claim 4, 5, 11 or 13, wherein the soluble amounts of the supporting solute in the ion exchange liquid membrane, the catholyte and the anolyte are independently not less than 0.1 mol/L.

**18.** The flow battery according to claim 4, 5, 11 or 13, wherein

the cation of the supporting solute is one or more selected from $H^+$, $NH_4^+$, $Li^+$, $Na^+$, $K^+$, $Zn^{2+}$, $Ca^{2+}$, $Mg^{2+}$, $EMIM^+$, $PMIM^+$, and $BMIM^+$,
the anion of the supporting solute is one or more selected from $OH^-$, $F^-$, $Cl^-$, Br, $I^-$, $PF_6^-$, $BF_4^-$, $TFSI^-$, $NO_3^-$, $HCO_3^-$, $ClO_4^-$, $SO_4^{2-}$, $PO_4^{3-}$, and $OTf^-$.

**19.** The flow battery according to claim 4, 5, 11 or 13, wherein the supporting solute is one or more selected from KCl, $NH_4Cl$, LiCl, NaCl, $CaCl_2$, $MgCl_2$, EMIMCl, PMIMCl, BMIMCl, $LiNO_3$, LiBr, EMIMBr, PMIMBr, BMIMBr, KI, EMIMI, PMIMI, BMIMI, $LiPF_6$, $NaPF_6$, $LiBF_4$, $NaBF_4$, LiTFSI, $LiClO_4$, and LiOTf.

**20.** The flow battery according to claim 4, 5, 11 or 13, wherein the functional additive is selected from a salting-out functional additive; a pH adjustment functional additive; and a functional additive to expand the electrochemical stability window, to adjust the density of the electrolyte or to adjust the solubility of the active material.

**21.** A battery stack comprising at least one ion exchange liquid membrane flow battery according to any one of claims 1 to 20.

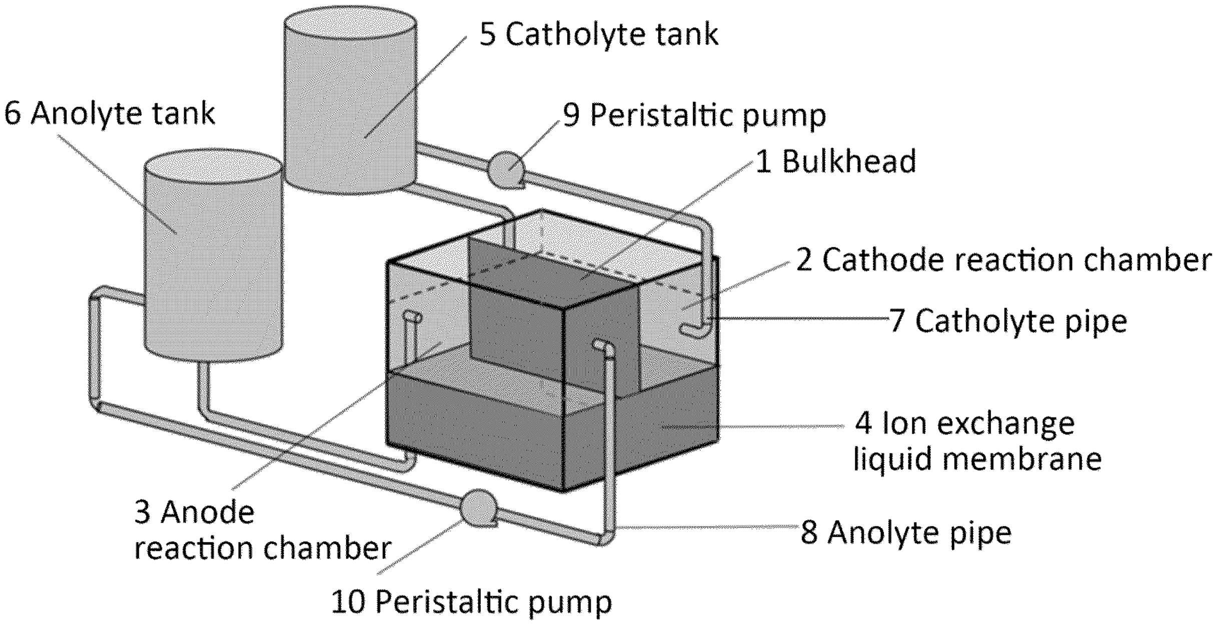
5 Catholyte tank
6 Anolyte tank
9 Peristaltic pump
1 Bulkhead
2 Cathode reaction chamber
7 Catholyte pipe
4 Ion exchange liquid membrane
3 Anode reaction chamber
8 Anolyte pipe
10 Peristaltic pump

Fig.1

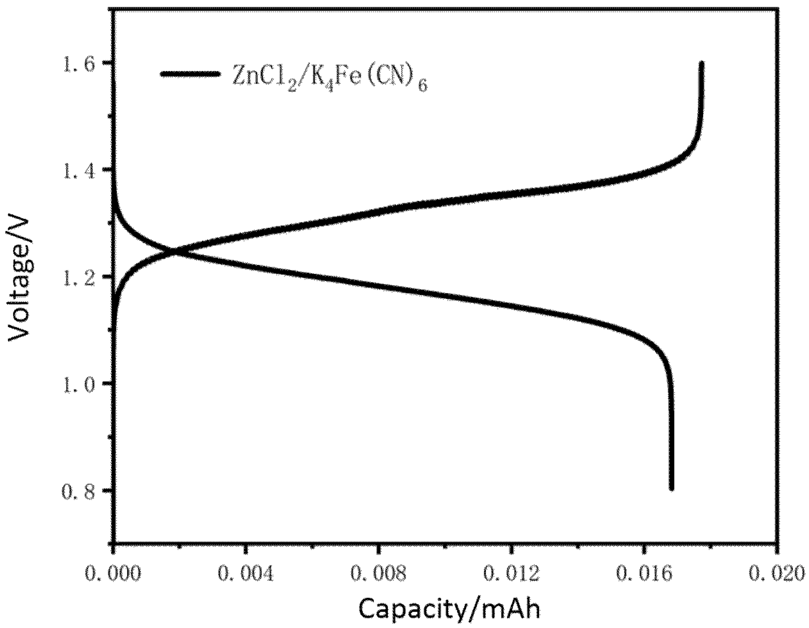
$ZnCl_2/K_4Fe(CN)_6$
Voltage/V
1.6
1.4
1.2
1.0
0.8
0.000
0.004
0.008
0.012
0.016
0.020
Capacity/mAh

Fig.2

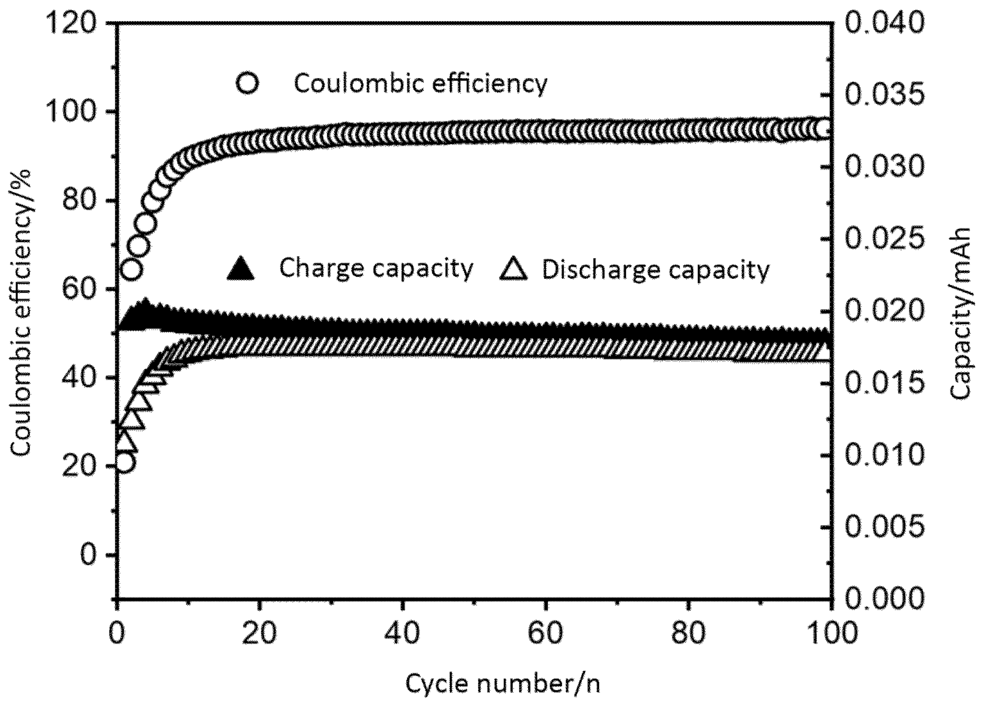
120
100
80
60
40
20
0
0.040
0.035
0.030
0.025
0.020
0.015
0.010
0.005
0.000
0
20
40
60
80
100
Coulombic efficiency
Charge capacity
Discharge capacity
Coulombic efficiency/%
Capacity/mAh
Cycle number/n

Fig.3

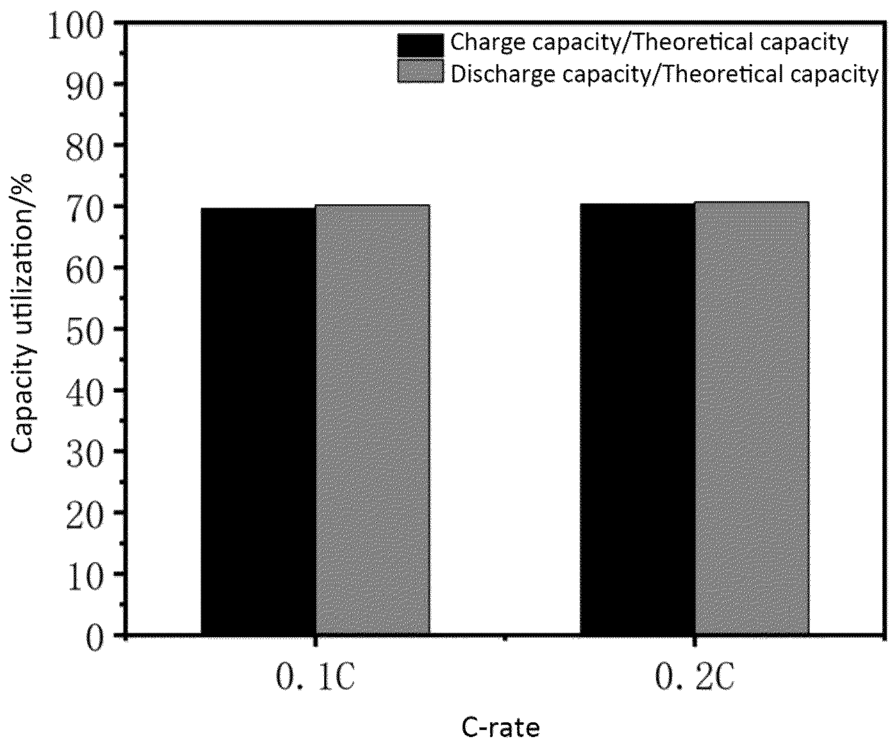
100
90
80
70
60
50
40
30
20
10
0
Charge capacity/Theoretical capacity
Discharge capacity/Theoretical capacity
Capacity utilization/%
0. 1C
0. 2C
C-rate

Fig.4

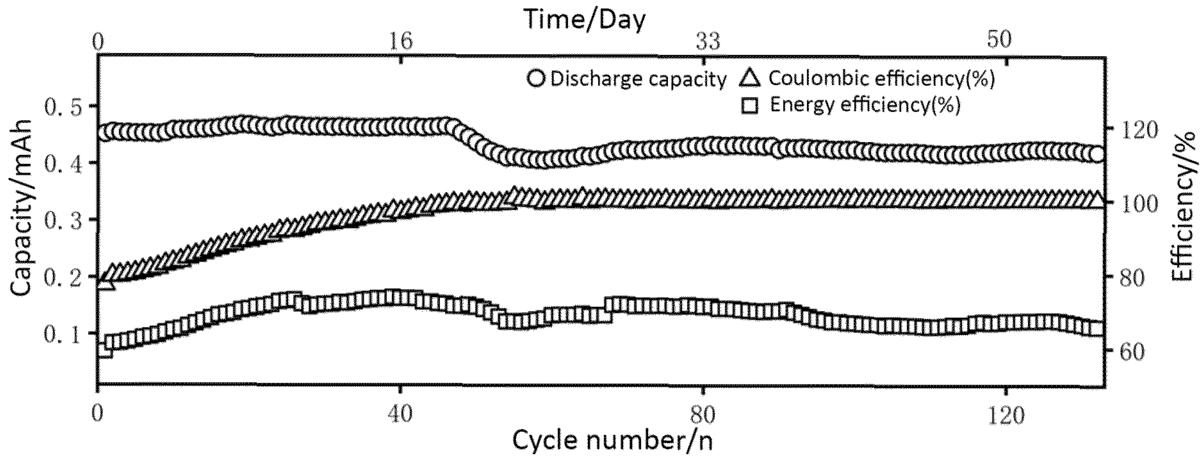
Time/Day
0
16
33
50
Discharge capacity
Coulombic efficiency(%)
Energy efficiency(%)
Capacity/mAh
0.5
0.4
0.3
0.2
0.1
Efficiency/%
120
100
80
60
0
40
80
120
Cycle number/n

Fig.5

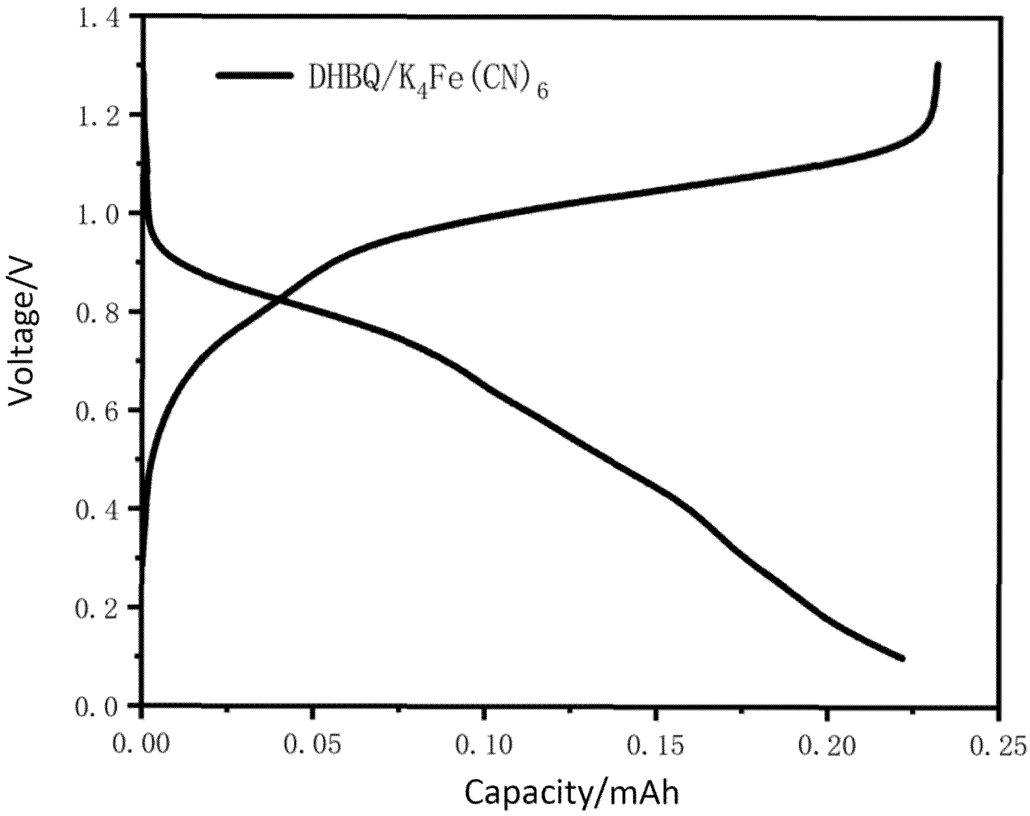
DHBQ/$K_4Fe(CN)_6$
Voltage/V
1.4
1.2
1.0
0.8
0.6
0.4
0.2
0.0
0.00
0.05
0.10
0.15
0.20
0.25
Capacity/mAh

Fig.6

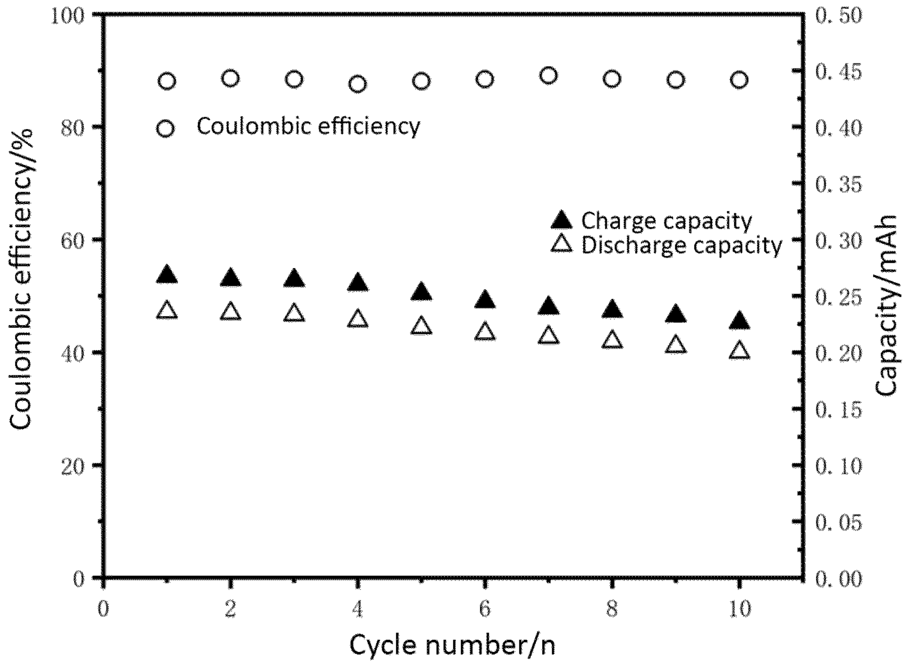
Coulombic efficiency
Charge capacity
Discharge capacity
Coulombic efficiency/%
Capacity/mAh
Cycle number/n
100
80
60
40
20
0
0.50
0.45
0.40
0.35
0.30
0.25
0.20
0.15
0.10
0.05
0.00
0
2
4
6
8
10

Fig.7

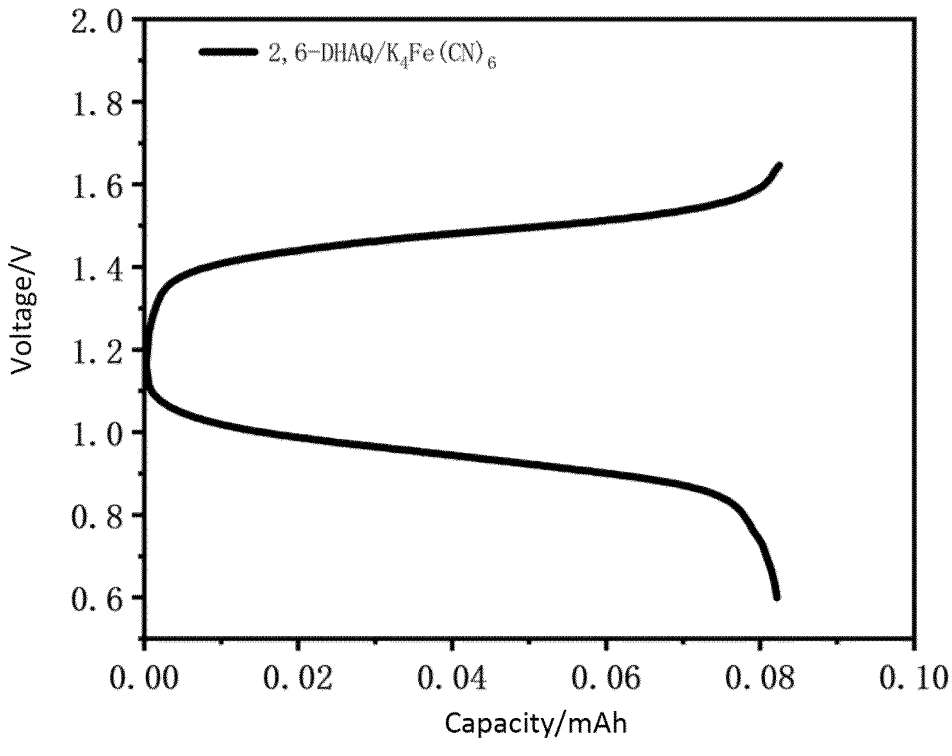
2,6-DHAQ/K4Fe(CN)6
Voltage/V
Capacity/mAh
2.0
1.8
1.6
1.4
1.2
1.0
0.8
0.6
0.00
0.02
0.04
0.06
0.08
0.10

Fig.8

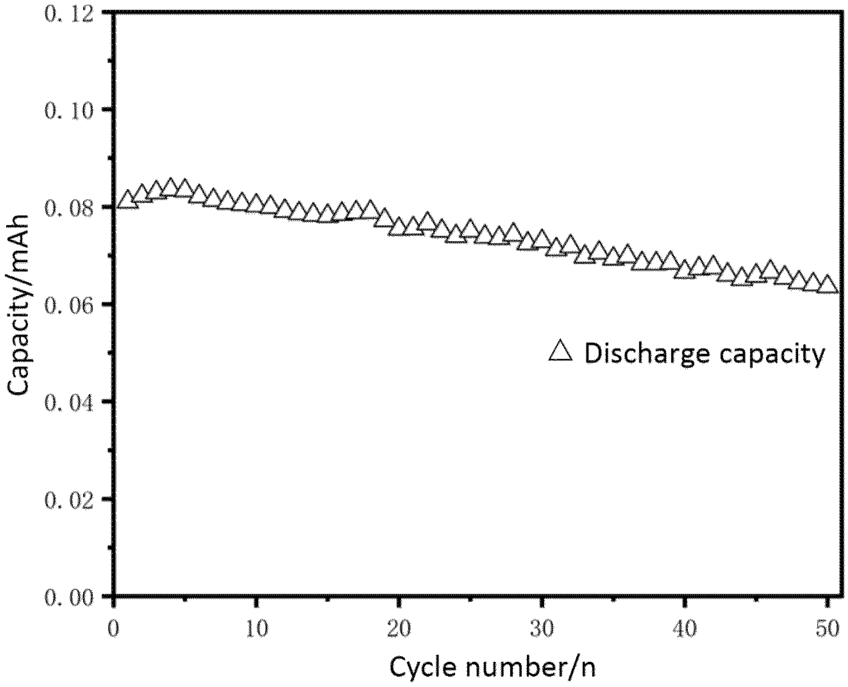
0.12
0.10
0.08
0.06
0.04
0.02
0.00
Capacity/mAh
△ Discharge capacity
0
10
20
30
40
50
Cycle number/n

Fig.9

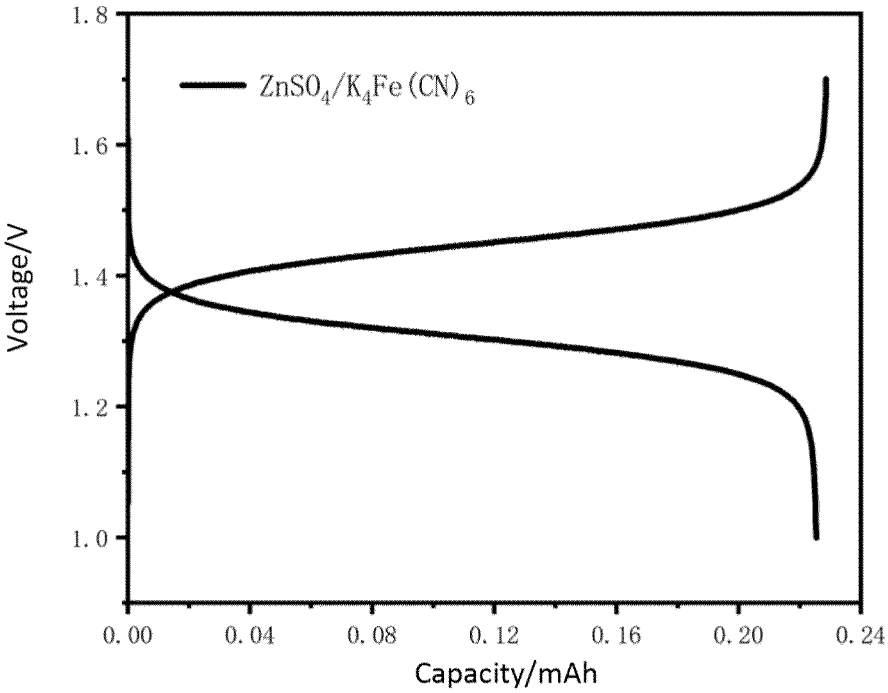
1.8
1.6
1.4
1.2
1.0
Voltage/V
$ZnSO_4/K_4Fe(CN)_6$
0.00
0.04
0.08
0.12
0.16
0.20
0.24
Capacity/mAh

Fig.10

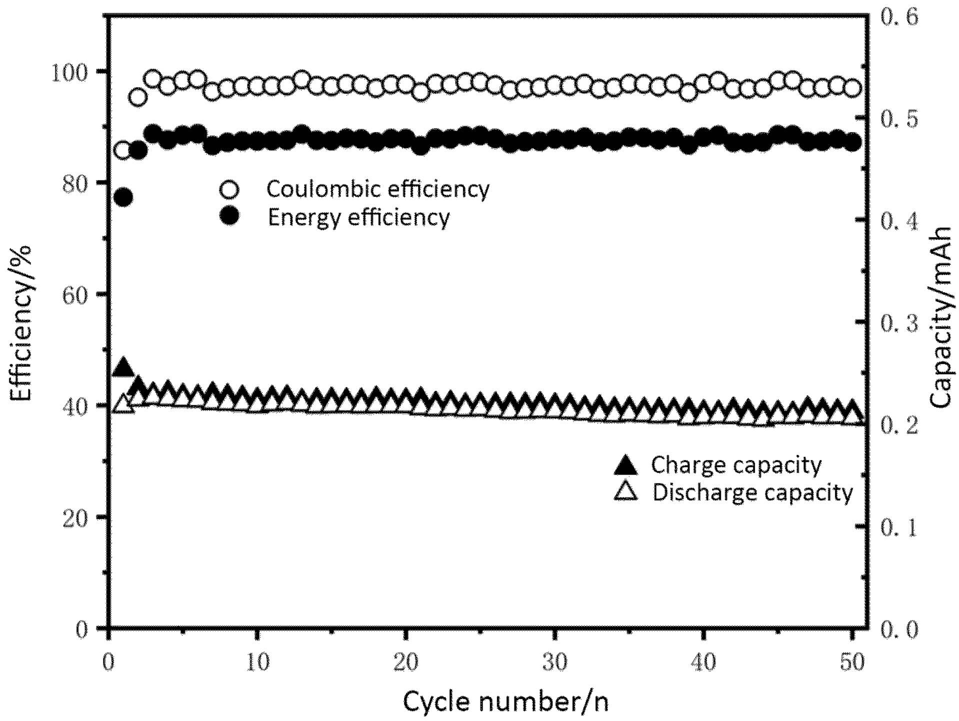
Efficiency/%
Capacity/mAh
Cycle number/n
Coulombic efficiency
Energy efficiency
Charge capacity
Discharge capacity
0
20
40
60
80
100
0. 0
0. 1
0. 2
0. 3
0. 4
0. 5
0. 6
0
10
20
30
40
50

Fig.11

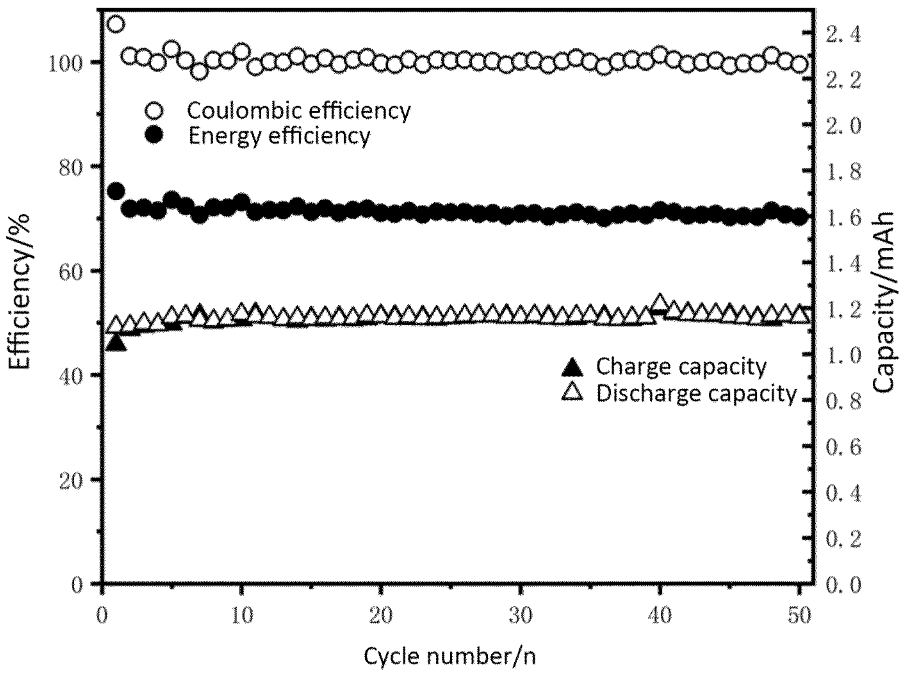
Efficiency/%
Capacity/mAh
Cycle number/n
Coulombic efficiency
Energy efficiency
Charge capacity
Discharge capacity
0
20
40
60
80
100
0. 0
0. 2
0. 4
0. 6
0. 8
1. 0
1. 2
1. 4
1. 6
1. 8
2. 0
2. 2
2. 4
0
10
20
30
40
50

Fig.12

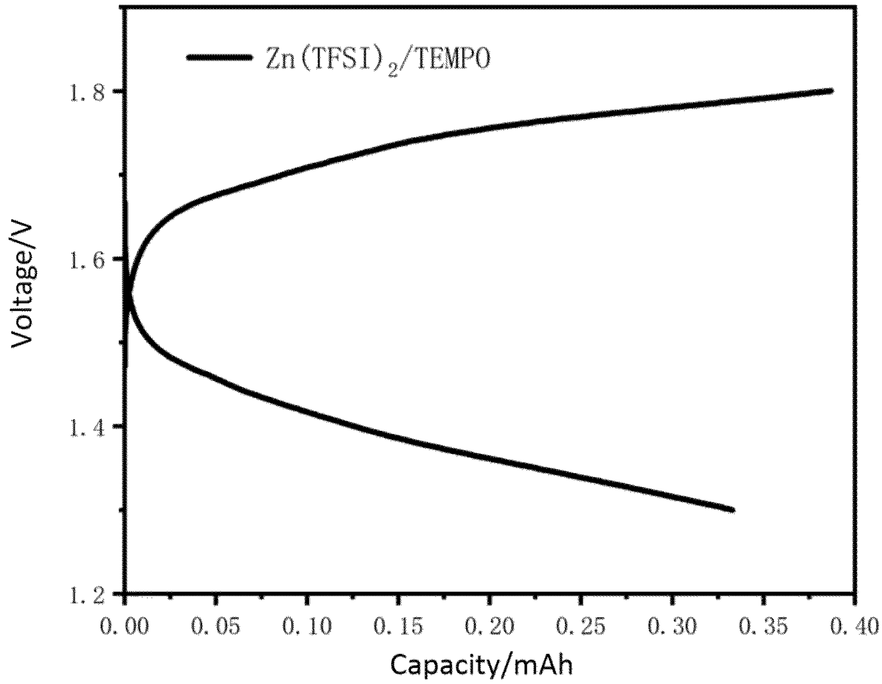
Zn(TFSI)$_2$/TEMPO
Voltage/V
1.8
1.6
1.4
1.2
0.00
0.05
0.10
0.15
0.20
0.25
0.30
0.35
0.40
Capacity/mAh

Fig.13

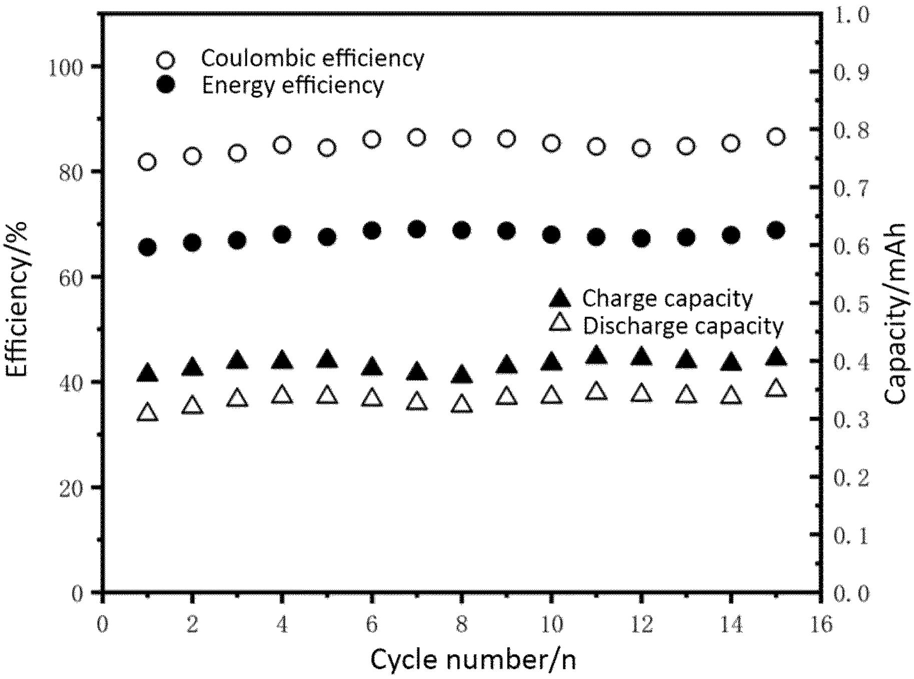
Coulombic efficiency
Energy efficiency
Charge capacity
Discharge capacity
Efficiency/%
Capacity/mAh
Cycle number/n

Fig.14

## INTERNATIONAL SEARCH REPORT

International application No.
**PCT/CN2022/125238**

**A. CLASSIFICATION OF SUBJECT MATTER**

H01M 8/18(2006.01)i

According to International Patent Classification (IPC) or to both national classification and IPC

**B. FIELDS SEARCHED**

Minimum documentation searched (classification system followed by classification symbols)

H01M

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 电池, 液流, 正极, 阴极, 负极, 阳极, 电解液, 电解质溶液, 液体, 液态, 分隔, 隔开, 间隔, 分层, 密度, 离子液体, battery, cell, flow, positive, cathode, negative, anode, electrolyte, liquid, separate, spacing, stratify, density, ionic, liquid

**C. DOCUMENTS CONSIDERED TO BE RELEVANT**

| Category* | Citation of document, with indication, where appropriate, of the relevant passages | Relevant to claim No. |
|---|---|---|
| Y | CN 109599577 A (CHINA SALT JINTAN CO., LTD.) 09 April 2019 (2019-04-09)<br>description, paragraphs 0007-0072, and figure 1 | 1-21 |
| Y | CN 106025249 A (YUNNAN KEWEI LIQUID METAL VALLEY R&D CO., LTD.) 12 October 2016 (2016-10-12)<br>description, paragraphs 0004-0033, and figure 1 | 1-21 |
| Y | CN 102498589 A (MASSACHUSETTS INSTITUTE OF TECHNOLOGY) 13 June 2012 (2012-06-13)<br>description, paragraphs 0029-0044, and figure 1 | 1-21 |
| Y | CN 112500438 A (CHINASALT JINTAN CO., LTD. et al.) 16 March 2021 (2021-03-16)<br>description, paragraphs 0004-0046 | 1-21 |
| Y | CN 111244518 A (DALIAN INSTITUTE OF CHEMICAL PHYSICS, CHINESE ACADEMY OF SCIENCES) 05 June 2020 (2020-06-05)<br>description, paragraphs 0004-0027 | 1-21 |
| A | US 2012169298 A1 (BOULDER IONICS CORP.) 05 July 2012 (2012-07-05)<br>entire document | 1-21 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 December 2022** | **22 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/125238**

| Patent document cited in search report | | | Publication date (day/month/year) | Patent family member(s) | | | Publication date (day/month/year) |
|---|---|---|---|---|---|---|---|
| CN | 109599577 | A | 09 April 2019 | None | | | |
| CN | 106025249 | A | 12 October 2016 | None | | | |
| CN | 102498589 | A | 13 June 2012 | WO | 2011011056 | A2 | 27 January 2011 |
| | | | | US | 2011014505 | A1 | 20 January 2011 |
| | | | | EP | 2457274 | A2 | 30 May 2012 |
| | | | | CA | 2767920 | A1 | 27 January 2011 |
| | | | | US | 2015303525 | A1 | 22 October 2015 |
| | | | | US | 2018294533 | A1 | 11 October 2018 |
| | | | | JP | 2012533865 | A | 27 December 2012 |
| | | | | EP | 2709188 | A1 | 19 March 2014 |
| CN | 112500438 | A | 16 March 2021 | None | | | |
| CN | 111244518 | A | 05 June 2020 | None | | | |
| US | 2012169298 | A1 | 05 July 2012 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PAULA NAVALPOTRO et al.** A membrane-free redox flow battery with two immiscible redox Electrolytes. *Angew. Chem. Int. Ed.,* 2017, vol. 56, 12460-12465
- **GUODONG LI et al.** membrane-free Zn/MnO2 flow battery for large-scale energy storage. *Adv. Energy Mater.,* 2020, 1902085
- **JINTAO MENG et al.** A stirred self-stratified battery for large-scale energy storage. *Joule,* 15 April 2020, vol. 4, 953-966
- Electrolyte Lifetime in Aqueous Organic Redox Flow Batteries: A Critical Review. *Chemical Reviews,* 2020, vol. 120 (14), 6467-6489
- Recent developments in organic redox flow batteries: A critical review. *Journal of Power Sources,* 2017, vol. 360 (1), 243-283
- Recent progress in organic redox flow batteries: Active materials, electrolytes and membranes. *Journal of Energy Chemistry,* vol. 2018 (S2095495617311051
- Molecular redox species for next-generation batteries. *Chemical Society Reviews,* 2021, vol. 50, 5863-5883
- Recent progress in organic redox flow batteries: Active materials, electrolytes and membranes. *Journal of Energy Chemistry,* vol. 2018, S2095495617311051
- Critical Appraisal of Salting-Out and Its Implications for Chemical and Biological Sciences. *Chemical Reviews,* 2005, vol. 105 (1), 1-10
- Salting out of proteins using ammonium sulfate precipitation. *Methods in enzymology,* 2014, vol. 541 (541), 85-94
- Mechanism of protein salting in and salting out by divalent cation salts: balance between hydration and salt binding. *Biochemistry,* 1984, vol. 23 (25), 5912-5923
- Salting-In and Salting-Out of Water-Soluble Polymers in Aqueous Salt Solutions. *Journal of Physical Chemistry B,* 2012, vol. 116 (17), 5234-5241
- Electrolyte Lifetime in Aqueous Organic Redox Flow Batteries: A Critical Review. *Chemical Reviews,* 2020
- Molecular redox species for next-generation batteries. *Chemical Society Reviews,* 2021